# EUROPEAN PATENT APPLICATION

(11) **EP 2 423 225 A2**
(43) Date of publication of application: **29.02.2012**
(21) Application number: 10176103.9
(22) Date of filing: 17.09.2004
(51) Int. Cl.: C07K 16/18

(54) **NOGO-A binding molecules and pharmaceutical use thereof**

(30) Priority: 19.09.2003 GB 0321997
(62) Divisional of application: 10160905.5
(71) Applicant: Novartis AG, 4056 Basel (CH); Novartis Pharma GmbH, 1230 Wien (AT); University of Zürich, 8006 Zürich (CH)
(72) Inventor: Barske, Carmen, 79540, Lörrach (DE); Frentzel, Stefan, 79539, Lörrach (DE); Mir, Anis Khusro, 68870, Bartenheim (FR); Schwab, Martin, 8057, Zürich (CH); Vitaliti, Alessandra, 4104, Oberwil (CH)
(74) Representative: Roth, Peter Richard

(57) **Abstract**

The present invention provides an antibody which is capable of binding to the human NogoA_342-357 epitope defined by amino acid sequence NQQELPTALTKLVKED, the use of such an antibody as a pharmaceutical, especially in the treatment of nerve repair; a pharmaceutical composition comprising said antibody; and a method of treatment of diseases associated with nerve repair.

## Description

This invention relates to NogoA binding molecules, such as for example monoclonal antibodies or Fab fragments thereof.

Neuronal regeneration following injury in the adult central nervous system (CNS) is limited due to the presence of the inhibitory myelin environment that ensheaths axons and formation of scar tissue. In the last few years important insights have been gained into the molecular understanding why the CNS is unable to spontaneously repair itself following injury. Inhibitory molecules in the myelin are the major impediment for the axonal regeneration, particularly immediately after the injury. So far NogoA, Myelin-Associated Glycoprotein (MAG) and myelin-oligodendrocyte glycoprotein (OMgp) have been characterised as potent inhibitors of neurite outgrowth. In addition, myelin also contains other inhibitory components, such as, chondroitin sulphate proteoglycans. Nogo-A is a member of the reticulon protein family and it has at least two biologically active and pharmacologically distinct domains termed Amino-Nogo and Nogo-66. While the receptor site for the former is not known so far, Nogo-66 inhibits neuronal growth in vitro and in vivo via the neuronal receptor NgR. In addition to Nogo-66, MAG and OMgp also bind to the NgR with high affinity and inhibit neurite outgrowth.

Potential new research approaches currently pursued for enhancement of nerve repair include digestion of scar tissue using an enzyme chondroitinase ABC, bridging techniques using Olfactory ensheathing cells and stem cells and protein growth factors to boost neuronal growth. Blocking actions of neurite outgrowth inhibitors by modulation of intracellular signalling mediators such as Rho , a membrane-bound guanosine trisphosphatase (GTPase), which appears to be a key link in the inhibition of axonal growth. Cyclic adenosine monophosphate (cAMP) which can overcome myelin associated inhibition in vitro and induce regeneration in vivo. Use of peptide inhibitor of the NgR receptor (NEP 1-40) to induce neuronal regrowth and functional recovery in rats following spinal injury.

In addition to the use of the approaches described above, attention has also focused upon the use of certain monoclonal antibodies to neutralize neurite growth inhibitory molecules of the central and peripheral nervous system, in particular to neutralize the neurite growth inhibitory activity of NogoA. Thus it has been shown that the monoclonal antibody IN-1 or the IN-1 Fab fragment thereof induce neurite outgrowth in vitro and enhance sprouting and regeneration in vivo (Schwab ME et al. (1996) Physiol. Rev. 76, 319-370). Testing different domains of the NogoA for neurite growth inhibitory acitvity have delineated several inhibitory domains in the molecule (Chen et al. (2000) Nature 403, 434-439; GrandPre eta 1. (2000) Nature 403, 439-444; Prinjha et al. (2000) Nature 403, 383-384.
Natural immunoglobulins or antibodies comprise a generally Y- shaped multimeric molecule having an antigen-binding site at the end of each upper arm. The remainder of the structure, in particular the stem of the Y mediates effector functions associated with the immunoglobulins. Antibodies consists of a 2 heavy and 2 light chains. Both heavy and light chains comprise a variable domain and a constant part. An antigen binding site consists of the variable domain of a heavy chain associated with the variable domain of a light chain. The variable domains of the heavy and light chains have the same general structure. More particularly, the antigen binding characteristics of an antibody are essentially determined by 3 specific regions in the variable domain of the heavy and light chains which are called hypervariable regions or complementarity determining regions (CDRs). These 3 hypervariable regions alternate with 4 framework regions (FRs) whose sequences are relatively conserved and which are not directly involved in binding. The CDRs form loops and are held in close proximity by the framework regions which largely adopt a β-sheet conformation. The CDRs of a heavy chain together with the CDRs of the associated light chain essentially constitute the antigen binding site of the antibody molecule. The determination as to what constitutes an FR or a CDR region is usually made by comparing the amino acid sequence of a number of antibodies raised in the same species. The general rules for identifying the CDR and FR regions are general knowledge of a man skilled in the art and can for example be found in the webside (http://www.bioinf.org.tik/abs/).

It has now surprisingly been found that a novel monoclonal human antibody (hereinafter called "3A6") raised in Medarex Mice (genetically reconstituted mice with human immunoglobulin genes) against human NiG and of the IgG type has better properties than the NogoA antibodies of the prior art (Schwab ME et al. (1996) Physiol. Rev. 76, 319-370), especially with regard to the binding affinity to NogoA of different species including the homo sapiens and with regard to its higher Nogo-A neurite outgrowth neutralizing activity at a given antibody concentration. Moreover it is now possible to construct other NogoA binding molecules having the same hypervariable regions as said antibody.

Accordingly, the invention provides binding molecules to a particular region or epitope of NogoA (hereinafter referred to as "the Binding Molecules of the invention" or simply "Binding Molecules"). Preferably, the Binding Molecules of the invention bind human NogoA_342-357 (epitope of 3A6 in human NiG; = SEQ ID NO: 6), human NogoA (SEQ ID NO: 5) or human NiG (which is the most potent neurite outgrowth inhibitory fragment of NogoA and starts at amino acid No. 186 and ends at amino acid No. 1004 of human NogoA, = SEQ ID NO: 5) with a dissociation constant (Kd) < 1000nM, more preferably with a Kd < 100 nM, most preferably with a Kd < 10 nM. The binding reaction may be shown by standard methods (qualitative assays) including, for example, the ELISA method described in Example 6 and the biosensor affinity method described in the Example 7. In addition, the binding to human NogoA and almost more importantly the efficiency may be shown in a neurite outgrowth assay, e.g. as described below.

Thus, in a further preferred embodiment the Binding Molecules (at a concentration of 100 µg/ml, preferably10 µg/ml, more preferably at 1.0 µg/ml even more preferably at 0.1 µg/ml) enhance the number of neurites of rat cerebellar granule cells on a substrate of monkey brain protein extract by at least 20%, preferably 50%, most preferred 80% compared to the number of neurites of rat cerebellar granule cells which are treated with a control antibody that does not bind to the human NogoA, human NiG or NogoA_342-357 polypeptide (i.e. that has a dissociation constant > 1000 nM).

In a further preferred embodiment the Binding Molecules of the invention comprises at least one antigen binding site, said antigen binding site comprising in sequence, the hypervariable regions CDR-H1-3A6, CDR-H2-3A6 and CDR-H3-3A6; said CDR-H1-3A6 having the amino acid sequence SEQ ID NO: 8, said CDR-H2-3A6 having the amino acid sequence SEQ ID NO: 9, and said CDR-H3-3A6 having the amino acid sequence SEQ ID NO: 10; and direct equivalents thereof.

In a further aspect of the invention, the Binding Molecule of the invention comprises at least one antigen binding site, said antigen binding site comprising either
a) in sequence the hypervariable regions CDR-H1-3A6, CDR-H2-3A6 and CDR-H3-3A6; said CDR-H1-3A6 having the amino acid sequence of SEQ ID NO: 8, said CDR-H2-3A6 having the amino acid sequence of SEQ ID NO: 9, and said CDR-H3-3A6 having the amino acid sequence SEQ ID NO: 10; or
b) in sequence the hypervariable regions CDR-L1-3A6, CDR-L2-3A6 and CDR-L3-3A6, said CDR-L1-3A6 having the amino acid sequence of SEQ ID NO: 11, said CDR-L2-3A6 having the amino acid sequence of SEQ ID NO: 12, and said CDR-L3-3A6 having the amino acid sequence of SEQ ID NO: 13; or
c) direct equivalents thereof.

In a further aspect of the invention, the Binding Molecule of the invention comprises at least
a) a first domain comprising in sequence the hypervariable regions CDR-H1-3A6, CDR-H2-3A6 and CDR-H3-3A6; said CDR-H1-3A6 having the amino acid sequence of SEQ ID NO: 8, said CDR-H2-3A6 having the amino acid sequence of SEQ ID NO: 9, and said CDR-H3-3A6 having the amino acid sequence SEQ ID NO: 10; and
b) a second domain comprising in sequence the hypervariable regions CDR-L1-3A6, CDR-L2-3A6 and CDR-L3-3A6, said CDR-L1-3A6 having the amino acid sequence of SEQ ID NO: 11, said CDR-L2-3A6 having the amino acid sequence of SEQ ID NO: 12, and said CDR-L3-3A6 having the amino acid sequence of SEQ ID NO: 13; or
c) direct equivalents thereof.

Moreover, the invention also provides the following Binding Molecule of the invention, which comprises at least one antigen binding site comprising
a) either the variable part of the heavy chain of 3A6 (SEQ ID NO: 2); or
b) the variable part of the light chain of 3A6 (SEQ ID NO: 3), or direct equivalents thereof.

When the antigen binding site comprises both the first and second domains, these may be located on the same polypeptide molecule or, preferably, each domain may be on a different chain, the first domain being part of an immunoglobulin heavy chain or fragment thereof and the second domain being part of an immunoglobulin light chain or fragment thereof.

Examples of Binding Molecules of the invention include antibodies as produced by B-cells or hybridomas and human or chimeric or humanized antibodies or any fragment thereof, e.g. F(ab')₂; and Fab fragments, as well as single chain or single domain antibodies.

A single chain antibody consists of the variable domains of an antibody heavy and light chains covalently bound by a peptide linker usually consisting of from 10 to 30 amino acids, preferably from 15 to 25 amino acids. Therefore, such a structure does not include the constant part of the heavy and light chains and it is believed that the small peptide spacer should be less antigenic than a whole constant part. By "chimeric antibody" is meant an antibody in which the constant regions of heavy or light chains or both are of human origin while the variable domains of both heavy and light chains are of non- human (e.g. murine) origin. By "humanized antibody" is meant an antibody in which the hypervariable regions (CDRs) are of non-human (e.g. murine) origin, while all or substantially all the other parts of the immunoglobulin e.g. the constant regions and the highly conserved parts of the variable domains, i.e. the framework regions, are of human origin. A humanized antibody may however retain a few amino acids of the murine sequence in the parts of the framework regions adjacent to the hypervariable regions.

Hypervariable regions may be associated with any kind of framework regions, preferably of murine or human origin. Suitable framework regions are described in "Sequences of proteins of immunological interest", Kabat E.A. et al, US department of health and human services, Public health service, National Institute of Health. Preferably the constant part of a human heavy chain of the Binding Molecules may be of the IgG4 type, including subtypes, preferably the constant part of a human light chain may be of the κ or λ type, more preferably of the κ type.

Monoclonal antibodies raised against a protein naturally found in all humans may be developed in a non-human system e. g. in mice. As a direct consequence of this, a xenogenic antibody as produced by a hybridoma, when administered to humans, elicits an undesirable immune response, which is predominantly mediated by the constant part of the xenogenic immunoglobulin. This clearly limits the use of such antibodies as they cannot be administered over a prolonged period of time. Therefore it is particularly preferred to use single chain, single domain, chimeric or humanized antibodies which are not likely to elicit a substantial allogenic response when administered to humans.

In view of the foregoing, a more preferred Binding Molecule of the invention is selected from a chimeric antibody, which comprises at least
a) one immunoglobulin heavy chain or fragment thereof which comprises (i) a variable domain comprising in sequence the hypervariable regions CDR-H1-3A6, CDR-H2-3A6 and CDR-H3-3A6 and (ii) the constant part or fragment thereof of a human heavy chain; said CDR-H1-3A6 having the amino acid sequence (SEQ ID NO: 8), said CDR-H2-3A6 having the amino acid sequence (SEQ ID NO: 9), and said CDR-H3-3A6 having the amino acid sequence (SEQ ID NO: 10), and
b) one immunoglobulin light chain or fragment thereof which comprises (i) a variable domain comprising in sequence the hypervariable regions CDR-L1-3A6, CDR-L2-3A6 and CDR-L3-3A6 and (ii) the constant part or fragment thereof of a human light chain; said CDR-L1-3A6 having the amino acid sequence (SEQ ID NO: 11), said CDR-L2-3A6 having the amino acid sequence (SEQ ID NO: 12), and said CDR-L3-3A6 having the amino acid sequence (SEQ ID NO: 13); or
direct equivalents thereof.

Alternatively, a Binding Molecule of the invention may be selected from a single chain binding molecule which comprises an antigen binding site comprising
a) a first domain comprising in sequence the hypervariable CDR-H1-3A6, CDR-H2-3A6 and CDR-H3-3A6; said CDR-H1-3A6 having the amino acid sequence (SEQ ID NO: 8), said CDR-H2-3A6 having the amino acid sequence (SEQ ID NO: 9), and said CDR-H3-3A6 having the amino acid sequence (SEQ ID NO: 10); and
b) a second domain comprising in sequence the hypervariable CDR-L1-3A6, CDR-L2-3A6 and CDR-L3-3A6; said CDR-L1-3A6 having the amino acid sequence (SEQ ID NO: 11), said CDR-L2-3A6 having the amino acid sequence (SEQ ID NO: 12), and said CDR-L3-3A6 having the amino acid sequence (SEQ ID NO: 13); and
c) a peptide linker which is bound either to the N- terminal extremity of the first domain and to the C-terminal extremity of the second domain or to the C-terminal extremity of the first domain and to the N-terminal extremity of second domain;
or direct equivalents thereof.

As it is well known, minor changes in an amino acid sequence such as deletion, addition or substitution of one or several amino acids may lead to an allelic form of the original protein which has substantially identical properties. Thus, by the term "direct equivalents thereof" is meant either any single domain Binding Molecule of the invention (molecule X)
(i) in which each of the hypervariable regions CDR-H1, CDR-H2, and CDR-H3 of the Binding Molecule is at least 50 or 80% homologous, preferably at least 90% homologous, more preferably at least 95, 96, 97, 98, 99% homologous to the equivalent hypervariable regions of CDR-H1-3A6 (SEQ ID NO: 8), CDR-H2-3A6 (SEQ ID NO: 9) and CDR-H3-3A6 (SEQ ID NO: 10), whereas CDR-H1 is equivalent to CDR-H1-3A6, CDR-H2 is equivalent to CDR-H2-3A6, CDR-H3 is equivalent to CDR-H3-3A6; and
(ii) which is capable of binding to the human NogoA, human NiG, or human NogoA_342-357, preferably with a dissociation constant (Kd) < 1000nM, more preferably with a Kd < 100 nM, most preferably with a Kd < 10 nM, or
   any binding molecule of the invention having at least two domains per binding site (molecule X')
(iii) in which each of the hypervariable regions CDR-H1, CDR-H2, CDR-H3, CDR-L1, CDR-L2 and CDR-L3 is at least 50 or 80% homologous, preferably at least 90% homologous, more preferably at least 95, 96, 97, 98, 99% identical to the equivalent hypervariable regions of CDR-H1-3A6 (SEQ ID NO: 8), CDR-H2-3A6 (SEQ ID NO: 9), CDR-H3-3A6 (SEQ ID NO: 10), CDR-L1-3A6 (SEQ ID NO: 11), CDR-L2-3A6 (SEQ ID NO: 12), and CDR-L3-3A6 (SEQ ID NO: 13), whereas CDR-H1 is equivalent to CDR-H1-3A6, CDR-H2 is equivalent to CDR-H2-3A6, CDR-H3 is equivalent to CDR-H3-3A6, CDR-L1 is equivalent to CDR-L1-3A6, CDR-L2 is equivalent to CDR-L2-3A6, CDR-L3 is equivalent to CDR-L3-3A6; and
(iv) which is capable of binding the human NogoA, human NiG, or human NogoA_342-357, preferably with a dissociation constant (Kd) < 1000nM, more preferably with a Kd < 100 nM, most preferably with a Kd < 10 nM.

Thus further embodiments of the inventions are for example a Binding Molecule which is capable of binding to the human NogoA, human NiG, *or human NogoA_342-357* with a dissociation constant < 1000nM and comprises at least one antigen binding site, said antigen binding site comprising either
- in sequence the hypervariable regions CDR-H1, CDR-H2, and CDR-H3, of which each of the hypervariable regions are at least 50%, preferably 80, 90, 95, 96, 97, 98, 99% homologous to their equivalent hypervariable regions CDR-H1-3A6 (SEQ ID NO: 8), CDR-H2-3A6 (SEQ ID NO: 9) and CDR-H3-3A6 (SEQ ID NO: 10); or
- in sequence the hypervariable regions CDR-L1, CDR-L2, and CDR-L3, of which each of the hypervariable regions are at least 50%, preferably 80, 90, 95, 96, 97, 98, 99% homologous to their equivalent hypervariable regions CDR-L1-3A6 (SEQ ID NO: 11), CDR-L2-3A6 (SEQ ID NO: 12) and CDR-L3-3A6 (SEQ ID NO: 13).

Furthermore, a Binding Molecule which is capable of binding the human NogoA, human NiG, or human NogoA_342-357 with a dissociation constant < 1000nM and comprises
- first antigen binding site comprising in sequence the hypervariable regions CDR-H1, CDR-H2, and CDR-H3, of which each of the hypervariable regions are at least 50%, preferably 80, 90, 95, 96, 97, 98, 99% homologous to their equivalent hypervariable regions CDR-H1-3A6 (SEQ ID NO: 8), CDR-H2-3A6 (SEQ ID NO: 9) and CDR-H3-3A6 (SEQ ID NO: 10); and
- a second antigen binding site comprising in sequence the hypervariable regions CDR-L1, CDR-L2, and CDR-L3, of which each of the hypervariable regions are at least 50%, preferably 80, 90, 95, 96, 97, 98, 99% homologous to their equivalent hypervariable regions CDR-L1-3A6 (SEQ ID NO: 11), CDR-L2-3A6 (SEQ ID NO: 12) and CDR-L3-3A6 (SEQ ID NO: 13).

This dissociation constant may be conveniently tested in various assays including, for example, the biosensor affinity method described in Example 7. In addition, the binding and functional effect of the Binding Molecules may be shown in a bioassay, e.g. as described below.

The constant part of a human heavy chain may be of the y1; y2; y3; y4; α1; α2; δ or ε type, preferably of the γ type, more preferably of the y4; type, whereas the constant part of a human light chain may be of the κ or λ type (which includes the λ1; λ2; and λ3 subtypes) but is preferably of the κ type. The amino acid sequence of all these constant parts are given in Kabat et al (Supra).

Conjugates of the binding molecules of the invention, e. g. enzyme or toxin or radioisotope conjugates, are also included within the scope of the invention.

"Polypeptide", if not otherwise specified herein, includes any peptide or protein comprising amino acids joined to each other by peptide bonds, having an amino acid sequence starting at the N-terminal extremity and ending at the C-terminal extremity. Preferably, the polypeptide of the present invention is a monoclonal antibody, more preferred is a chimeric (also called V-grafted) or humanised (also called CDR-grafted) monoclonal antibody. The humanised (CDR-grafted) monoclonal antibody may or may not include further mutations introduced into the framework (FR) sequences of the acceptor antibody.

A functional derivative of a polypeptide as used herein includes a molecule having a qualitative biological activity in common with a polypeptide to the present invention, i.e. having the ability to bind to the human NogoA, human NiG, or human NogoA_342-357. A functional derivative includes fragments and peptide analogs of a polpypeptide according to the present invention. Fragments comprise regions within the sequence of a polypeptide according to the present invention, e.g. of a specified sequence. The term "derivative" is used to define amino acid sequence variants, and covalent modifications of a polypeptide according to the present invention. e.g. of a specified sequence. The functional derivatives of a polypeptide according to the present invention, e.g. of a specified sequence, e.g. of the hypervariable region of the light and the heavy chain, preferably have at least about 65%, more preferably at least about 75%, even more preferably at least about 85%, most preferably at least about 95, 96, 97, 98, 99% overall sequence homology with the amino acid sequence of a polypeptide according to the present invention, e.g. of a specified sequence, and substantially retain the ability to bind the human NogoA, human NiG or human NogoA_342-357.

The term "covalent modification" includes modifications of a polypeptide according to the present invention, e.g. of a specified sequence; or a fragment thereof with an organic proteinaceous or non-proteinaceous derivatizing agent, fusions to heterologous polypeptide sequences, and post-translational modifications. Covalent modified polypeptides, e.g. of a specified sequence, still have the ability bind to the human NogoA, human NiG or human NogoA_342-357 by crosslinking. Covalent modifications are traditionally introduced by reacting targeted amino acid residues with an organic derivatizing agent that is capable of reacting with selected sides or terminal residues, or by harnessing mechanisms of post-translational modifications that function in selected recombinant host cells. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deaminated under mildly acidic conditions. Other post-translational modifications include hydroxylation of proline and lysine, phosphorylation of hydroxyl groups of seryl, tyrosine or threonyl residues, methylation of the α-amino groups of lysine, arginine, and histidine side chains, see e.g. T. E. Creighton, Proteins: Structure and Molecular Properties, W. H. Freeman & Co., San Francisco, pp. 79-86 (1983). Covalent modifications e.g. include fusion proteins comprising a polypeptide according to the present invention, e.g. of a specified sequence and their amino acid sequence variants, such as immunoadhesins, and N-terminal fusions to heterologous signal sequences.

"Homology" with respect to a native polypeptide and its functional derivative is defined herein as the percentage of amino acid residues in the candidate sequence that are identical with the residues of a corresponding native polypeptide, after aligning the sequences and introducing gaps, if necessary, to achieve the maximum percent homology, and not considering any conservative substitutions as part of the sequence identity. Neither N- or C-terminal extensions nor insertions shall be construed as reducing identity or homology. Methods and computer programs for the alignment are well known.

"Amino acid(s)" refer to all naturally occurring L-α-amino acids, e.g. and including D-amino acids. The amino acids are identified by either the well known single-letter or three-letter designations.

The term "amino acid sequence variant" refers to molecules with some differences in their amino acid sequences as compared to a polypeptide according to the present invention, e.g. of a specified sequence. Amino acid sequence variants of a polypeptide according to the present invention, e.g. of a specified sequence, still have the ability to bind to human NogoA or human NiG or more preferably to NogoA_342-357. Substitutional variants are those that have at least one amino acid residue removed and a different amino acid inserted in its place at the same position in a polypeptide according to the present invention, e.g. of a specified sequence. These substitutions may be single, where only one amino acid in the molecule has been substituted, or they may be multiple, where two or more amino acids have been substituted in the same molecule. Insertional variants are those with one or more amino acids inserted immediately adjacent to an amino acid at a particular position in a polypeptide according to the present invention, e.g. of a specified sequence. Immediately adjacent to an amino acid means connected to either the α-carboxy or α-amino functional group of the amino acid. Deletional variants are those with one or more amino acids in a polypeptide according to the present invention, e.g. of a specified sequence, removed. Ordinarily, deletional variants will have one or two amino acids deleted in a particular region of the molecule.

A binding molecule of the invention may be produced by recombinant DNA techniques. In view of this, one or more DNA molecules encoding the binding molecule must be constructed, placed under appropriate control sequences and transferred into a suitable host organism for expression.

In a very general manner, there are accordingly provided
(i) DNA molecules encoding a single domain Binding Molecule of the invention, a single chain Binding Molecule of the invention, a heavy or light chain or fragments thereof of a Binding Molecule of the invention; and
(ii) the use of the DNA molecules of the invention for the production of a Binding Molecule of the invention by recombinant means.

The present state of the art is such that the skilled person will be able to synthesize the DNA molecules of the invention given the information provided herein i.e. the amino acid sequences of the hypervariable regions and the DNA sequences coding for them. A method for constructing a variable domain gene is for example described in EP 239 400 and may be briefly summarized as follows: A gene encoding a variable domain of a monoclonal antibody of whatever specificity is cloned. The DNA segments encoding the framework and hypervariable regions are determined and the DNA segments encoding the hypervariable regions are removed so that the DNA segments encoding the framework regions are fused together with suitable restriction sites at the junctions. The restriction sites may be generated at the appropriate positions by mutagenesis of the DNA molecule by standard procedures. Double stranded synthetic CDR cassettes are prepared by DNA synthesis according to the sequences given CDR-H1-3A6, CDR-H2-3A6, CDR-H3-3A6, CDR-L1-3A6, CDR-L2-3A6 and CDR-L3-3A6 above. These cassettes are provided with sticky ends so that they can be ligated at the junctions to the framework by standard protocol for achieving a DNA molecule encoding an immunoglobulin variable domain.

Furthermore, it is not necessary to have access to the mRNA from a producing hybridoma cell line in order to obtain a DNA construct coding for the monoclonal antibodies of the invention. Thus PCT application W0 90/07861 gives full instructions for the production of a monoclonal antibody by recombinant DNA techniques given only written information as to the nucleotide sequence of the gene.

The method comprises the synthesis of a number of oligonucleotides, their amplification by the PCR method, and their splicing to give the desired DNA sequence.

Expression vectors comprising a suitable promoter or genes encoding heavy and light chain constant parts are publicly available. Thus, once a DNA molecule of the invention is prepared it may be conveniently transferred in an appropriate expression vector.

DNA molecules encoding single chain antibodies may also be prepared by standard methods, for example, as described in W0 88/1649.

In a particular embodiment of the invention, the recombinant means for the production of some of the Binding Molecules of the invention includes first and second DNA constructs as described below:

The first DNA construct encodes a heavy chain or fragment thereof and comprises
a) a first part which encodes a variable domain comprising alternatively framework and hypervariable regions, said hypervariable regions comprising in sequence DNA-CDR-H1-3A6 (SEQ ID NO: 14), DNA-CDR-H2-3A6 (SEQ ID NO: 15) and DNA-CDR-H3-3A6 (SEQ ID NO: 16); this first part starting with a codon encoding the first amino acid of the variable domain and ending with a codon encoding the last amino acid of the variable domain, and
b) a second part encoding a heavy chain constant part or fragment thereof which starts with a codon encoding the first amino acid of the constant part of the heavy chain and ends with a codon encoding the last amino acid of the constant part or fragment thereof, followed by a non-sense codon.

Preferably, the second part encodes the constant part of a human heavy chain, more preferably the constant part of the human γ4 chain. This second part may be a DNA fragment of genomic origin (comprising introns) or a cDNA fragment (without introns).

The second DNA construct encodes a light chain or fragment thereof and comprises
a) a first part which encodes a variable domain comprising alternatively framework and hypervariable regions; said hypervariable regions comprising in sequence DNA-CDR-L1-3A6 (SEQ ID NO: 17), DNA-CDR-L2-3A6 (SEQ ID NO: 18) and DNA-CDR-L3-3A6 (SEQ ID NO: 19), this first part starting with a codon encoding the first amino acid of the variable domain and ending with a codon encoding the last amino acid of the variable domain, and
b) a second part encoding a light chain constant part or fragment thereof which starts with a codon encoding the first amino acid of the constant part of the light chain and ends with a codon encoding the last amino acid of the constant part or fragment thereof followed by a non-sense codon.

Preferably, the second part encodes the constant part of a human light chain, more preferably the constant part of the human κ chain.

The first or second DNA construct advantageously comprises a third part which is located upstream of the first part and which encodes part of a leader peptide; this third part starting with the codon encoding the first amino acid and ending with the last amino acid of the leader peptide. This peptide is required for secretion of the chains by the host organism in which they are expressed and is subsequently removed by the host organism. Preferably, the third part of the first DNA construct encodes a leader peptide having an amino acid sequence substantially identical to the amino acid sequence of the heavy chain leader sequence as shown in SEQ ID NO: 21 (starting with the amino acid at position -19 and ending with the amino acid at position -1). Also preferably, the third part of the second DNA construct encodes a leader peptide having an amino acid sequence as shown in SEQ ID NO: 23 (light chain, starting with the amino acid at position -18 and ending with the amino acid at position -1).

Each of the DNA constructs are placed under the control of suitable control sequences, in particular under the control of a suitable promoter. Any kind of promoter may be used, provided that it is adapted to the host organism in which the DNA constructs will be transferred for expression. However, if expression is to take place in a mammalian cell, it is particularly preferred to use the promoter of an immunoglobulin gene.

The desired antibody may be produced in a cell culture or in a transgenic animal. A suitable transgenic animal may be obtained according to standard methods which include micro injecting into eggs the first and second DNA constructs placed under suitable control sequences transferring the so prepared eggs into appropriate pseudo- pregnant females and selecting a descendant expressing the desired antibody.

When the antibody chains have to be produced in a cell culture, the DNA constructs must first be inserted into either a single expression vector or into two separate but compatible expression vectors, the latter possibility being preferred.

Accordingly, the invention also provides an expression vector able to replicate in a prokaryotic or eukaryotic cell line which comprises at least one of the DNA constructs above described.

Each expression vector containing a DNA construct is then transferred into a suitable host organism. When the DNA constructs are separately inserted on two expression vectors, they may be transferred separately, i.e. one type of vector per cell, or co- transferred, this latter possibility being preferred. A suitable host organism may be a bacterium, a yeast or a mammalian cell line, this latter being preferred. More preferably, the mammalian cell line is of lymphoid origin e.g. a myeloma, hybridoma or a normal immortalized B-cell, but does not express any endogeneous antibody heavy or light chain.

It is also preferred that the host organism contains a large number of copies of the vectors per cell. If the host organism is a mammalian cell line, this desirable goal may be reached by amplifying the number of copies according to standard methods. Amplification methods usually consist of selecting for increased resistance to a drug, said resistance being encoded by the expression vector.

In another aspect of the invention, there is provided a process for producing a multi-chain binding molecule of the invention, which comprises (i) culturing an organism which is transformed with the first and second DNA constructs of the invention and (ii) recovering an active binding molecule of the invention from the culture.

Alternatively, the heavy and light chains may be separately recovered and reconstituted into an active binding molecule after in vitro refolding. Reconstitution methods are well-known in the art; Examples of methods are in particular provided in EP 120 674 or in EP 125 023. Therefore a process may also comprise
(i) culturing a first organism which is transformed with a first DNA construct of the invention and recovering said heavy chain or fragment thereof from the culture and
(ii) culturing a second organism which is transformed with a second DNA construct of the invention and recovering said light chain or fragment thereof from the culture and
(iii) reconstituting in vitro an active binding molecule of the invention from the heavy chain or fragment thereof obtained in (i) and the light chain or fragment thereof obtained in (ii).

In a similar manner, there is also provided a process for producing a single chain or single domain binding molecule of the invention which comprises
(i) culturing an organism which is transformed with a DNA construct respectively encoding a single chain or single domain binding molecule of the invention and
(ii) recovering said molecule from the culture.

The binding molecules of the invention exhibit very good nerve regeneration activity as shown, for example, in the granule cell neurite outgrowth model.

### 1. Granule cell neurite outgrowth assay (in vitro)

Brain tissue (cortex and brain stem) is taken and for each assay protein extract freshly prepared as described previously (Spillmann et al. 1998, Identification and characterization of a bovine neurite growth inhibitor (bNI-220), J Biol Chem. 1998 Jul 24;273(30):19283-93). A piece of frozen tissue (e.g. 0.25g) is homogenized in 3-4 Vol of 60mM Chaps - 20mM Tris pH 8.0-1mM EDTA with Protease blocker (10µg/ml Aprotinin - 5µg/ml, Leupeptin - 1µg/ml Pepstatin - 1 mM PMSF) at 4°C. Homogenate is put on a rotator at 4°C for 30min and centrifuged at 1 00'000g 45min 4°C in a TLA 100.3 rotor (Beckman TL-100ultracentrifuge). From supernatant the protein concentration is determined using BioRad.
Cerebellar granule cells are purified from trypsin dissociates of postnatal day 5-7 rat cerebellar tissue as described previously (Niederost et al 1999, Bovine CNS myelin contains neurite growth-inhibitory activity associated with chondroitin sulfate proteoglycans, J Neurosci. 1999 Oct 15;19(20):8979-89). The binding molecules of the invention are then preincubated for 30 min on the test substrate and removed before the cells are added. Cerebellar granule cells are added and incubated for 24 hours. To stop the experiment, 2 ml of 4 % buffered formaldehyde is slowly added to the culture dishes. Monkey brain membrane protein extract prepared as described above was adsorbed overnight at 15µg protein per cm² culture dish on Greiner 4-well dishes (Greiner, Nuertingen, Germany). Dishes are washed three times with warm Hank's solution before plating the neurons. Postnatal day (5-7) rat cerebellar granule cells are prepared as described above and plated at 50,000 cells/cm². Cells are cultured for 24 hr in serum-free medium, fixed, and immunostained with neurite marker MAB 1 b (Chemicon monoclonal Ab, 1:200). For the staining of cell bodies DAPI (4',6-diamidino-2-phenyl-indole, dihydrochloride, from Molecular Probes) is used after staining with MAB1b. For antibody experiments, the anti-Nogo-A mAbs or control IgG Ab are preincubated on the dishes for 30 min and subsequently removed.
Four fields at a defined distance to the edge of the well are randomly sampled for each well using a 40 X objective by counting all intersections of neurites with a line placed through the center of the observation field. All cell bodies touching the line are also counted, and an index ratio of neurites per cell body is calculated for each well as reported previously (Simonen et al, 2003, Neuron 38,201-211). All counts are done blindly on coded experiments and expressed as an index of neuritis per cell body. Results are expressed as mean index neuritis / cell body.

Enhancement of neurite outgrowth of cerebellar granule cell in the non-permissive environment of the above prepared spinal cord extract by preincubation with a binding molecule of the invention may be observed. E.g. a typical profile for the neutralizing effect of the human 3A6-IgGl and IgG4 antibody in the granule cell neurite outgrowth model is given below:

| | | Index Neurites / cell body | | %increase compared to control |
|---|---|---|---|---|
| IgG | | | | |
| no antibody | | 0.87 | | |
| +Control IgG | | 0.90 | | |
| 3AIgGI | 0.1 µg/ml | | 1.44 | 60% |
| 3A6 IgG4 | 0.1 µg/ml | | 1.43 | 59% |
| +Control IgG | | 0.92 | | |
| 3A6IgGI | 10.0 µg/ml | | 1.69 | 84% |
| 3A6 IgG4 | 10.0 µg/ml | | 1.55 | 68% |

The neutralizing activity of the molecules of the invention can also be estimated by measuring the regenerative sprouting and neurite outgrowth and functional recovery in the *in vivo* spinal cord injury models briefly described below.

### 2. Spinal cord injury models in rats and monkeys (in vivo)

Adult Lewis rats are injured microsurgically by transecting the dorsal half of the spinal cord bilaterally at the level of the 8^{th} thoracic vertebra. Laminectomy, anesthesia and surgery are described in Schnell and Schwab 1993 (Eur.J. Neurosci. 5: 1156 - 1171). *Neuroanatomical tracing:* The motor and sensory corticospinal tract is traced by injecting the anterograde tracer biotin dextran amine (BDA) into the cortex of the side opposite to the pump or the graft. BDA is transported to the spinal cord within 10 - 14 days and visualized using diaminobenzidine (DAB) as a substrate as described in Brösamle et al., (2000 J.Neurosci. 20: 8061-8068).
Two weeks after a spinal cord injury destroying about 40 % of the spinal cord segment T8, mainly in the dorsal half, including both main CSTs: tracing of the CST in control animals show a moderate degree of reactive sprouting of the tract. This phenomenon corresponds to the spontaneous sprouting in response to injury well known in the literature. Injured rats being treated with the binding molecules of the invention or with pumps delivering the binding molecules of the invention may show an enhanced sprouting at the lesion site and regeneration of damaged axons neurite outgrowth of damaged neurites. Moreover the animals may show improved recovery of sensorimotor functions. Such functional tests are described previously (Merkler et al, 2001, J. Neuroscience 21,3665-73).

### 3. Tissue Distribution of Antibodies in Adult Monkey CNS

The antibody 3A6 is purified as IgG and concentrated to 3 mg/ml in PBS. Mouse serum derived IgG (Chemicon Int., Temecula/CA, USA) or a mAB directed against wheat auxin (AMS Biotechnology, Oxon/UK) are used as control treatments. Two male adult macaque monkeys (Macaca fascicularis) are used in this study for intrathecal infusion.

### Surgical procedures

Anaesthesia is induced by intramuscular injection of ketamine (Ketalar®; Parke-Davis, 5 mg/kg, i.m.). Atropine is injected i.m. (0.05 mg/kg) to reduce bronchial secretions. An intravenous catheter is placed in the femoral vein for continuous perfusion with a mixture of propofol 1% (Fresenius ®) and glucose 4% solution (1 volume of Propofol and 2 volumes of glucose solution), inducing a deeper anaesthesia. The animal is then placed in a stereotaxic framework. Under sterile conditions, a vertical midline skin incision is performed from C2 to Th1. The fascia cut and the spinal processes of C2 to Th1 are exposed. The paravertebral muscles are retracted and the laminae of C6, C7 and Th1 dissected. A complete C6 laminectomy and an upper C7 hemilaminectomy are then performed. The dura mater is exposed and incised longitudinally above the 7^{th} and the 8^{th} cervical spinal segments, corresponding to the rostral zone of the spinal portion covered by the 6^{th} cervical lamina. A polyethylene tube (10 cm long), connected to an osmotic pump (Alzet®, 2ML1; flow: 50µg/hr) delivering the hNogo-A antibody, is inserted below the dura and pushed a few millimeter rostrally and attached to the dura with a suture. The osmotic pump is placed and secured in a cavity made in the mass of back muscles a few centimeter lower than the laminectomy, on the left side. The tube is secured along its trajectory with sutures to muscle tissue. The muscles and the skin are sutured and the animal recovered from anaesthesia usually 15-30 minutes after interruption of the venous perfusion with propofol. The animal is treated postoperatively with an antibiotic (Ampiciline 10%, 30 mg/kg, s.c.). Additional doses of Carprofen are given daily during one week.

The monkeys are sacrificed 8 days after implantation of the osmotic pump. Sedation is first induced with ketamine, as mentioned above, followed by a deep anaesthesia obtained by i.p. injection of a lethal dose of pentobarbital (90 mg/kg). The animals are perfused transcardially with 0.4 litre of 0.9% saline, followed by 4 litres of fixative (4% solution of paraformaldehyde in 0.1 M phosphate buffer, pH=7.6). Perfusion is continued with 3 solutions of sucrose of increasing concentration (10% in fixative, 20 and 30 % in phosphate buffer).

### Histological procedures, immuno-fluorescence and -histochemistry

Brains and spinal cords of the monkeys are carefully dissected, cryo-protected in 30% sucrose and sectioned at 40 µm in a cryostate. For detection of infused mABs an anti-human secondary antibody is used (Jackson Laboratories). For double labelling, the following antibodies can be used: the rabbit AS472 (affinity purified) for endogenous Nogo-A (Chen, 2000), rabbit antibodies against GFAP for astrocytes, and a rabbit antibody against Cathepsin D (DAKO) for lysosomal localization. All the antisera are visualized by TRITC or FITC coupled corresponding secondary antibodies, or using the ABC-DAB system (Vector). Sections are analysed by epifluorescence on a Zeiss Axiophot or by confocal microscopy (ZEISS LSM 410).

The spinal cords are analysed at the infusion site and 6 cm caudal to it. High levels of 3A6 are present at the infusion site. In the more caudal spinal cord, central canal and cord surface are strongly labelled, whereas grey and white matter show a more homogenous labelling, which, however, is specific and clearly over background. A similar situation is present in the forebrain with strong labelling of surface and ventricles and good penetration of the Nogo-A antibody into the parenchyma.

These experiments show that spinal intrathecal infusion of antibodies against a CNS cell surface antigen lead to a good distribution of the antibody through the CSF circulation in the inner (ventricles, central canal) and outer liquor spaces. The IgG antibodies penetrate well into the brain and spinal cord tissue. Whereas the control IgG is washed out rapidly, the antibody against Nogo-A are retained in the tissue.

### 4. Tests for nerve repair and functional improvement in spinal lesions in monkeys

Anaesthesia is induced by intramuscular injection of ketamine (Ketalar®; Parke-Davis, 5 mg/kg, i.m.). Atropine is injected i.m. (0.05 mg/kg) to reduce bronchial secretions. An intravenous catheter is placed in the femoral vein for continuous perfusion with a mixture of propofol 1% (Fresenius ®) and glucose 4% solution (1 volume of Propofol and 2 volumes of glucose solution), inducing a deeper anaesthesia. The animal is then placed in a stereotaxic framework. Under sterile conditions, a vertical midline skin incision is performed from C2 to Th1. The fascia cut and the spinal processes of C2 to Th1 are exposed. The paravertebral muscles are retracted and the laminae of C6, C7 and Th1 dissected. A complete C6 laminectomy and an upper C7 hemilaminectomy are then performed. In order to deliver the molecules in close proximity of the lesion, the free tip of a polyethylene tube attached to the pump is fixed under the dura a few millimeter rostrally to the lesion.

Behavioural manual dexterity tests can be performed according to the published procedure. Manual dexterity is trained by placing the monkey seated in a primate chair in front of a Perspex modified "Brinkman board" (10 cm x 20 cm) containing 50 holes randomly distributed; 25 holes being oriented horizontally and 25 vertically {Liu, 1999 15428 /id;Rouiller, 1998 13239 /id}. 2.7. The regeneration and sprouting of fibers can be assessed as described. The anterograde tracer injected in the right hemisphere is Biotinylated Dextran Amine (BDA, Molecular Probe®, 10% in saline). In the left hemisphere, the fluorescent anterograde tracer Fluorescein Dextran (Molecular Probe®, 10% in saline) is injected. Histological processing to visualise the tracers can be performed as described in details previously {Rouiller, 1994 8322 /id}.

Therefore the invention also provides
(i) the use of the binding molecules of the invention in the nerve repair of a mammalian nervous system, in particular human nervous system,
(ii) a method of repairing nerves of a mammalian nervous system, in particular human nervous system which comprises administering an effective amount of the binding molecules of the invention to a patient in need of such treatment, or
(iii) a pharmaceutical composition for nerve repair of a mammalian nervous system, in particular human nervous system which comprises the binding molecules of the invention and a pharmaceutically acceptable carrier or diluent.

In particular, the binding molecules of the invention are useful for axonal regeneration and improved sprouting after nerve fiber damage. Thus the molecules of the invention have a wide utility in particular for human subjects. For example the binding molecule of the invention are useful in the treatment of various diseases of the peripheral (PNS) and central (CNS) nervous system, i.e. more particularly in neurodegenerative diseases such as Alzheimer disease, Parkinson disease, Amyotrophic lateral sclerosis (ALS), Lewy like pathologies or other dementia in general, diseases following cranial, cerebral or spinal trauma, stroke or a demyeliating disease. Such demyelinating diseases include, but are not limited to, multiple sclerosis, monophasic demyelination, encephalomyelitis, multifocal leukoencephalopathy, panencephalitis, Marchiafava-Bignami disease, pontine myelmolysis, adrenoleukodystrophy, Pelizaeus-Merzbacher disease, Spongy degeneration, Alexander's disease, Canavan's disease, metachromatic leukodystrophy and Krabbe's disease. In one example, administration of the binding molecules of the invention can be used to treat a demyelinating disease associated with NogoA protein. In another example, cells which express the binding molecules of the invention may be transplanted to a site spinal cord injury to facilitate axonal growth throughout the injured site. Such transplanted cells would provide a means for restoring spinal cord function following injury or trauma. Such cells could include olfactory ensheathing cells and stem cells of different lineages of fetal nerve or tissue grafts.

The effect of long-term delayed Nogo-A blockade on functional recovery and neuroanatomical plasticity in adult rats after stroke was the subject of an abstract published by Shih-Yen Tsai, Anay Pradham, Josh Rosales, Anis K. Mir, Martin E. Schwab, Gwendolyn L. Kartje in 2004. Purified anti-Amino Nogo-A antibody was administered by using osmotic pumps to adult rats 8 weeks after middle cerebral artery occlusion (MCAO). Recovery of function was examined using the skilled forelimb reaching test and the ladder rung walking test. The preliminary results showed that even when treating with anti-Amino Nogo-A blockade two months after stroke, recovery of function improved.

In addition, the Binding Molecules of the invention are useful for the treatment of degenerative ocular disorders which may directly or indirectly involve the degeneration of retinal or corneal cells including ischemic retinopathies in general, anterior ischemic optic neuropathy, all forms of optic neuritis, age-related macular degeneration, diabetic retinopathy, cystoid macular edema (CME), retinitis pigmentosa, Stargardt's disease, Best's vitelliform retinal degeneration, Leber's congenital amaurosis and other hereditary retinal degenerations, pathologic myopia, retinopathy of prematurity,and Leber's hereditary optic neuropathy, the after effects of corneal transplantation or of refractive corneal surgery, and herpes keratitis.

Furthermore, the Binding Molecules of the invention are useful for the treatment of psychiatric conditions, particularly schizophrenia and depression.

For these indications, the appropriate dosage will, of course, vary depending upon, for example, the particular molecule of the invention to be employed, the mode of administration and the nature and severity of the condition being treated. In general, the dosage preferably will be in the range of 1 µg/kg/day to 1 mg/kg/day. The Binding Molecules of the invention are conveniently administered by pumps or injected as therapeutics at the lesioned site, e.g. they can be administered directly into the CNS intracranially or into the spine intrathecally to the lesioned site.
The Binding Molecules of the invention can be provided alone, or in combination, or in sequential combination with other agents. For example, the binding molecules of the invention can be administered in combination with anti-inflammatorv agents such as but not limited to corticosteroids following stroke or spinal cord injury as a means for blocking further neuronal damage and inhibition of axonal regeneration, Neurotrophic factors such as NGF, BDNF or other drugs for neurodegenerative diseases such as Exelon™ or Levodopa. Other suitable combination partners for the treatment of stroke are Alteplase and Desmoteplase (DSPA, e.g. disclosed in WO90/09438). In one embodiment, the present invention provides a combination comprising a Binding Molecule of the invention and Desmoteplase, in particular for the treatment of stroke as well as pharmaceutical compositions comprising said combination. As used herein, two agents are said to be administered in combination when the two agents are administered simultaneously or are administered independently in a fashion such that the agents will act at the same time.

The structure of the active ingredients identified by code nos., generic or trade names may be taken from the actual edition of the standard compendium "The Merck Index" or from databases, e.g. Patents International (e.g. IMS World Publications) or other databases provide by IMS Health. The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

Pharmaceutical compositions of the invention may be manufactured in conventional manner. E.g. a composition according to the invention comprising the molecules of the invention is preferably provided in lyophilized form. For immediate administration it is dissolved in a suitable aqueous carrier, for example sterile water for injection or sterile buffered physiological saline.

To aid in making up suitable compositions, the binding molecules of the invention and optionally a second drug enhancing the effect of the Binding Molecules of the invention, may be packaged separately within the same container, with instructions for mixing or concomitant administration. Optional second drug candidates are provided above.

The synergistic effect of a combination of the binding molecules of the invention and growth factors such as NGF may be demonstrated in vivo by the spinal cord injury models.

The invention will be more fully understood by reference to the following examples. They should not, however, be construed as limiting the scope of the invention.

In the following examples all temperatures are in degree Celsius (°C).

The monoclonal antibody of attention in the Examples is a Binding Molecule according to the present invention comprising the variable part of the light chain (SEQ ID NO: 3) and the variable part of the heavy chain (SEQ ID NO: 2).

The following abbreviations are used:

| | |
|---|---|
| ELISA | enzyme linked immuno-sorbant assay |
| FACS | fluorescence activated cell sorting |
| FITC | fluorescein isothiocyanate |
| FBS | foetal bovine serum |
| HCMV | human cytomegalovirus promoter |
| IgG | immunoglobulin isotype G |
| MAb | monoclonal antibody |
| PBS | phosphate-buffered saline |
| PCR | polymerase chain reaction |

### Example 1: Methods:

*Generation* of *human Nogo-A expression constructs (pRK7-hNogo-A):* A human cDNA library constructed in lambda gt10 (Clontech) is screened with duplicate filter sets using standard procedures. Fragments of human Nogo-A are amplified by PCR from human whole brain cDNA (Clontech) using a standard protocol and subsequently cloned into pBluescript, digested and isolated, or used as screening probes directly. A 400bp Xhol/Smal fragment is used as 5' probe, the 3' probe is amplified with primers CA-NA-2F: 5'-AAG CAC CAT TGA ATT CTG CAG TTC C-3' (SEO ID NO: 29) and CA-NA-3R: 5'-AAC TGC AGT ACT GAG CTC CTC CAT CTG C-3' (SEO ID NO: 30). Positive clones are isolated, subcloned and sequence confirmed. To obtain a full length human Nogo-A cDNA, overlapping clones are assembled using an unique EcoRI restriction site in the human Nogo-A sequence and subcloned into Bluescript vector, named Pbsnogoa. To obtain pRK7-hNogo-A, the full length cDNA was inserted into the eukaryotic expression vector pRK-7 by directional cloning.

*Generation of human NiG (hNiG) expression plasmids (pET28a-hNiG) for bacterial production:* A hNiG encoding DNA fragment is subcloned into BamHI/Xhol of pET28a (Novagen), after PCR amplification of the respective coding region from Pbsnogoa, in frame with the N-terminal His- and T7-tag for bacterial expression, using primer sets: forward 5'-GTC GCG GAT CCA TGG AGA CCC TTT TTG CTC TTC-3' (SEQ ID NO: 31); reverse 5'-GTT CTC GAG TTA TGA AGT TTT ACT CAG-3' (SEQ ID NO: 32). The final plasmid is termed pET28a-hNiG. hNiG was then expressed in E.coli BL21 pRP by induction with 1 mM Isopropyl-beta-D-thiogalactopyranoside (IPGT).

*Generation of mouse NiG-exon3 (mNiG-exon3) expression plasmid:* The region encoding mouse exon 3 is amplified from mouse genome BAC template with primers: forward 5'-GTG CGG ATC CAT GGA TTT GAA GGA GCA GC-3' (SEQ ID NO: 33); reverse 5'-GTT TCT CGA GTG AAG TTT TAT TCA GCT C-3' (SEQ ID NO: 34) and subcloned into the BamHI/Xhol cloning sites of pET28a. The final plasmid construct is named pET28a-mNiG-exon3.

*Cloning of monkey NiG:* PolyA RNA is isolated from frozen monkey brain tissue and cDNA are synthesised using an oligo dT primer. Two overlapping fragments covering the 5' and the 3' region of the cDNA are amplified by PCR using sequence-specific primers and a proofreading enzyme. The primers are designed using the known sequence of the human NiG cDNA. For amplification of the 5' fragment the primers are 5'-TCCACCCCGGCCGCGCCCAA-3' (SEO ID NO: 35) and 5'-AATGATGGGCAAAGCTGTGCTG-3' (SEO ID NO: 36), for the 3'-fragment 5'-GGTACAAAGATTGCTTATGAAACA-3' (SEO ID NO: 37) and 5'-AGCAGGGCCAAGGCAATGTAGG-3' (SEQ ID NO: 38). The two fragments are then subcloned and for each fragment at least 4 independent clones were sequenced. The full length cDNA is assembled by overlapping PCR using the primers mentioned above and the resulting product is cloned and sequenced again.

Production of recombinant NogoNiG proteins as defined above: The bacterial Nogo-A-deletion library is expressed in Escherichia coli. Proteins are extracted either by repeated sonication in sonication buffer (20 mM Tris, 50 mM NaH₂PO₄ 100 mM NaCl, pH 8.0) with 0.75 mg/ml Lysozyme, by solubilisation with B-Per™ (Pierce) or with 8 M urea. NiG expressed with pelB-leader is obtained from the periplasmic space according to the Novagen protocol for periplasmic protein purification. Supernatants of pET28-constructs are purified using the CO²⁺-Talon™ Metal Affinity Resin (Clontech) in a batch procedure. 8 M urea and B-Per™ solubilised lysates are brought to non-denaturing conditions by increasingly substituting the buffer with sonication buffer during the resin-batch procedure. Proteins are eluted with 250 mM imidazole in sonication buffer on a gravity column (BioRad). NiG proteins are further purified by gel filtration on Superdex 200 HiLoad 16/60. Supernatants of pGEX-6P constructs are purified with G-sepharose column in a batch procedure according to manufacturer indications (Amersham Pharmacia). Cleavage of GST-Nogo-66 is done by incubating solubilised GST-Nogo-66 with PreScission protease and subsequent HPLC purification. Gel electroelution is performed by preparative SDS-PAGE of IMAC-purified recombinant Nogo and elution with BioRad Electro-Eluter into 50 mM Tris, pH 7.4, 100 mM NaCl, 0.2% (w/v) CHAPS for 1 hr at 250 mA and followed by 30 s of reversed electrode polarities. Protein concentrations of chromatography-purified proteins are determined using Pierce Coomassie Stain and BSA as standard protein. Protein concentrations of gel eluted proteins are estimated based on band intensity of silver-stained gels (Merril CR, Dunau ML, Goldman D (1981) A rapid sensitive silver stain for polypeptides in polyacrylamide gels. Analyt.Biochem. 110:201-207) with BSA as a standard.

### Example 2 : Generation of human 3A6-IgG mAb

Medarex Mice (Recombinantly reconstituted with human immunoglobulin genes) are immunised subcutaneously with human NiG, corresponding to a particular sequence in human Nogo-A. 3A6 monoclonal antibody was generated by standard hybridoma technology by fusion of the spleen cells of the mouse with a hybridoma cell line.
Immunisation of Medarex mice was carried out with with human NiG 70ug/mouse in the back of neck and flanks s.c.concentration 1.5mg in 1.9m1 Mixed V/V with TiterMax Adjuvant. Injection of 180µl s.c / mouse and subsequently boosted several times.
Determination of anti-Nogo-A Ab titers in serum with ELISA was carried out in 96 well plates were coated with 8ug/ml human NiG in PBS(100µl/well) Incubated 4 hours at room temperature (RT). Plates were flicked and refilled with 200µl /well blocking buffer(PBS+5% BSA), covered and incubated 1 h at RT or overnight at 4 degrees, then washed 4 times with tapwater, refilled with PBS and flicked. Mouse serum was diluted in PBS+10%FCS (100µl/well), and incubated 2h at RT or overnight at 4 degrees . Dilutions of mice sera used: 1:100,1:1000,1:10000, 1:30000.Wash step was repeated.Goat F(ab')2 anti-human IgG Fc specific HRP conjugate Ab was diluted in PBS/0.1%BSA /0.1 %Nonidet 40 (100 µl/well) and incubated 2h at RT or overnight at 4 degrees. Wash step was repeated. 100µl/well BM blue POD substrate were added and incubated in the dark at room temperature 15 minutes and 50µl/well 1 M H2SO4 was added to stop HPR substrate reaction. The O:D was determinated using a microplate reader set at 450nm. Screening of Hybridomas and clones with ELISA was carried out as described above. Human NiG( 8µg/ml, E.coli). IgG Isotyping with ELISA. Experiments were carried out to determine the IgG subclass of the antibodies. Plates were coated with human NiG and culture supernatants were used at dilutions of 1:10 to 1:100. The reactivity of the antibodies was evaluated by using a panel of mouse anti-human IgG subclass (IgG1, IgG2,IgG3,IgG4) HRP conjugated mAbs by incubation for 4h. anti-MCP1 IgG1 mAb was used as positive control. The Elisa was carried as described above. Generation of Hybridomas was done from mouse with the highest serum titers against human NiG in ELISA and was selected for fusion. Mouse was sacrificed by CO2 inhalation. Spleen taken aseptically and single cell suspension was made. Wash in PBS calcium ,magnesium free. Mouse myeloma cells(PAIO) were washed in PBS.
Equal numbers of mouse spleen cells 50 million were added with mouse myeloma cells and spin at RT for 10min. 900RPM. Supernatant withdrawn carefully and completely. Add dropwise 1 ml PEG 4000 as fusion agent (50:50 in PBS)under light agitation over 2-3 min. at RT. Shaken gently in water bath at 37degrees for 90 seconds. Add dropwise 5 to 10ml RPMI 1640 medium over 5 min. to dilute out the PEG, leave at RT for 10 min. Add another 20ml serum free medium and centrifuge. Resuspend in appropriate amount of HAT medium (RPMI+10%FCS+20ml/liter 50xHAT. Fused cells were plated out 100µl/well, into wells containing a feeder layer of peritoneal cells from Balb/c mice(1 ml/well).
Preparation of mouse peritoneal cells is carried out 24hours earlier and 1ml cultures in Hat medium, 24 wells Costar plate were prepared.The yield from one mouse is sufficient for one 24 well plate=approx. 2000 cells/well. Following sacrifice, the peritoneal cavity is washed out with 5ml of 0.34M sucrose, using a 10ml syringe and 18 gauge needle.Groups of 6 mice are collected into 1 tube,centrifuged,resuspended in HAT medium and aliqouted into wells. Culture medium was RPMI 1640 with Glutamax, containing 100µM hypoxanthine, 1.6µM Thymidine, 0.4µM Aminoptrin, 50µM beta-mercaptoethanol, 50µg/ml Gentamycin, 10% heat inactivated FCS. Medium was exchanged 50% every 3^{rd} or 4^{th} day depending on growth rate and appearance of hybridomas and after 14 days HAT medium was exchanged for HTmedium by leaving out Aminoptrin. Screening of hybridomas and clone generation Supernatants were tested when wells had reached approx. 80% confluency at a dilution of 1:3 in ELISA as described above. Single cell cloning was carried out from hybridomas that were positive for anti-Nogo Ab by limiting dilution. Cloning was carried out by limiting dilution, plating out 0.5 cells/100µl/well. 4x96,100µl wells are set up. Mouse PE cells were used for feeder layer. Clonal growth was checked microscopically, 100µl medium was added the day before screening is carried out.Growth rates of individual hybridomas vary, but ca. 10 days are needed for cultures to be dense enough, to yield sufficient antibody.
Screening of supernatants was done at dilution of 1:10,1:100. Culture expansion of positive clones was carried out and adapted to low serum conditions 1% for production in roller bottles and purification was done from culture supernatants in using protein A affinity.

### Example 3: Production and Purification of mouse 3A6 mAb and Fab 3A6:

Protein A Sepharose Cl-4B column was used (Pharmacia ; 11 cm bed height). Briefly, the culture supernatant after pH correction to 8.1 is loaded at 4 ml/min and the column washed to base-line at 8 ml/min using 100 mM Na₂HPO₄, pH 8.1. Bound material is finally eluted at 8 ml/min using 50 mM NaH₂PO₄ pH 3.0, 140 mM NaCl and immediately neutralized (pH 7.0) with 5 N NaOH and sterile filtered. Absorbance is monitored at 280 nm. Portion of the purified material are eventually further concentrated by ultrafiltration and/or dialyzed against PBS. All the buffers used in the purification are filtered on a 10 kDa ULTRASETTE™ tangential flow device (Filtron Technology Corporation) in order to remove possible endotoxin contaminations. For the same reason the Protein A resin is extensively washed with 20% ethanol and all tubings/pumps treated with 0.1 M NaOH prior to use. Protein concentration is measured spectrophotometrically at 280 nm using a reference absorption of 1.35 for 1 mg/ml. Purity is routinely assessed by SDS-PAGE under reducing conditions using 4-20% Novex gradient gels. Endotoxin content is measured by the classical Limulus Amoebocyte Lysate (LAL) reaction according to the manufacturer instructions (Endotell AG, Allschwil, Switzerland).

*Generation of F_{ab} fragments:* A portion of mouse 3A6 mAb is extensively dialyzed against 100 mM Na-actetate, pH 5.5, 2 mM EDTA and adjusted to a concentration of 6 mg/ml. F_{ab} fragments are generated by papain digestion (1:200 w/w ratio) in the presence of 0.25 mM cysteine. The reaction is allowed to proceed for 16 hours at 37 °C and then stopped by the addition of the specific papain inhibitor E64 (N-[N-(L-3-trans-carboxirane- 2-carbonyl)-L-leucyl]-agmatine) in large excess (10 µM). The digested antibody is then passed over a column of protein A Sepharose Fast Flow in order to remove intact material and Fc fragments. The F_{ab} fraction is extensively dialysed against PBS and concentrated to about 3 mg/ml. (Papain and E64 are from Roche Molecular Biochemicals).

### Example 4: HPLC, Mass Spectrometry and N-terminal amino acid sequencing of V_{L} and V_{H} region:

a) Reduction and Alkylation: Purified, dried 3A6 antibody are dissolved in 40 µl of 8M urea, 0.4M NH₄HCO₃, pH 8.3. 60 µg DTT (Calbiochem), pre-dissolved in 10µl of the same buffer as the protein, are added. Reduction is performed at 50°C for 30 min under argon (100 fold molar excess of DTT over protein thiols). After reduction, the sample is cooled to room temperature. 304 µl of iodoacetamide (Sigma Ultra, I-1149) dissolved in the same buffer as the protein is added. Carboxamidomethylation is carried out at room temperature for 15 min in the dark. 1 µl β-mercaptoethanol is added to quench the reaction.
b) Isolation of Heavy- and Light-Chain: Carboxamidomethylated heavy and light chains of antibody are isolated by Reverse Phase High Pressure Liquid Chromatography (RP-HPLC) on a Hewlett Packard 1090M HPLC System with DR5 pumping system and diode-array UV detector. The conditions for chromatography are: PerSeptive Biosystems Poros 2.1 x100 mm column packed with R1/H material; flow is 0.5 ml/min; solvents: (A) 0.1 % TFA in water and (B) 0.09% TFA / acetonitril/water 9:1; gradient 25-70% B in 8 minutes at 80°C; detection at 218 / 280 nm.
c) LC-ESI-MS: Mass spectrometry is carried out using a Q-Tof (Micromass, Manchester, UK) quadrupole time-of-flight hybrid tandem mass spectrometer equipped with a Micromass Z-type electrospray ionization source (ESI). Acquisition mass range is typically m/z 500-2000. Data are recorded and processed using MassLynx software. Calibration of the 500-2500 m/z scale is achieved by using the multiple-charged ion peaks of horse heart myoglobin (MW 16951.5).
d) HPLC-MS of heavy and light chain: Separation of reduced and carboxamidomethylated heavy and light chain is performed on a HP1100 HPLC system (Hewlett Packard, Palo-Alto, CA, USA) employing a 1 mmx150mm LC Packings column packed with Perseptive Biosystems POROS R1/H. The column is held at 60°C. Sample volumes of 10 µl are injected onto the column using a CTC PAL autosampler (CTC, Zwingen, Switzerland) fitted with a Valco model C6UW HPLC valve (Valco, Houston, TX, USA) and a 10 µl injection loop. HPLC was controlled by MassLynx software (Micromass, Manchester, UK). UV detection is at 214 nm. Eluent A is water containing 0.05% TFA. Eluent B is a 1:9 mixture of water: acetonitrile containing 0.045% TFA. A gradient from 20% B to 90% B is run in 20 minutes at 80 °C. The flow rate is typically 60 µl/min. The total flow from the LC system is introduced into the UV detection cell, then the ESI source without any splitting. The HPLC system is controlled and the signal from the UV detector is processed using MassLynx software (Micromass, Manchester, UK). The following 5 signals are detected

**Table 1:**

| **Measured**: | **Signal interpretation** |
|---|---|
| A= 50614.2 Da | H-Chain with carboxamidomethyl-cysteine (CAMCys) |
| B= 5077645 Da | Signal A+162 Da (= hexose) |
| E= 23727.8 Da | L-Chain with CAMCys |

d) N-terminal amino acid sequencing of V_{L} and V_{H} regions: Collected H+L chains peaks form HPLC are used for sequence analysis. Amino acid sequences are determined on a Hewlett Packard G1000A N-terminal Protein Sequencing System. The system performs automated Edman chemistry on protein samples retained on miniature adsorptive biphasic columns. An optimized chemistry method (double couple 3.0) is used to enhance chemical efficiency, minimize lags and herewith extend sequence analysis to about 50 residues. Analysis of PTH-amino acids is performed on an on-line Hewlett Packard HP1090 HPLC System equipped with a ternary pumping system and a narrowbore (2.1 mm x 25cm) PTH column.

### Results:

From mass analysis homogeneous heavy and light chain of mouse 3A6-IgG1 are determined. The H-chain is single glycosylated. Total mass analysis of heavy and light chain shows a single mass for both chains. HPLC chromatography of mouse 3A6-IgG1 shows a single peak. After HPLC purification followed by reduction and alkylation pure heavy and light chain are available. N-terminal sequence degradation is performed on light-chain and heavy-chain. Amino acids from the N-terminal sequence of L-chain and H-chain are identified by sequence degradation.
Light Chain
   **EIVLTQSPATLSLSPGERATLSCRASQSVS**
Heavy Chain
   **EVQLVESGGGLVQPGGSLRLSCAASGFTF**

### Example 5: Cloning of the heavy and light chain genes of human 3A6 mAb

Total RNA is prepared from 10⁷ hybridoma cells (clone 3A6) using TriPure reagent (Roche diagnostics, Germany, Cat.# 1667157) according to the manufacturers instructions. For cDNA synthesis, mRNA is isolated from above prepared total RNA using Oligotex Resin (Qiagen, Germany, cat. # 70022).

cDNA is generated by reverse transcription using the following conditions: 2 µl mRNA, 2 µl 10 x reverse transcription buffer, 2 µl (dT)₂₀ primer (10 µM), 0.5 µl RNasin (Promega, 40 U/ml), 2 µl dNTPs (5 mM each), 1 µl Omniscript™ reverse transcriptase (Qiagen, Cat # 205110), 10.5 µl ddH₂O, Reaction:1 hr at 37°C. For PCR amplification of cDNA encoding for the V_{H} and V_{L} the proofreading enzyme ProofStart™ DNA polymerase is used.

PCR of light and heavy chain: Reaction mix: 2 µl cDNA , 5 µl 10 x reaction buffer, 3 µl dNTPs (5 mM each), 2 µl 5'primer (10 µM) (see Table 2), 2µl 3'primer (10 µM) (see Table 2), 1 µl ProofStart (Qiagen, Cat # 202203), 36 µl ddH₂O. PCR conditions: 95°C/5 min, (95°C/40 sec, 53°C/1 min, 72°C 1 min) x 35, 72°C/10 min. The resulting PCR products are ligated directly into pCRbluntTOPO (Invitrogen). The ligation mix is transfected into TOP 10 cells (Invitrogen) and several clones are picked. The nucleotide sequences of the variable part of the heavy chain of the 3A6 mAb (V-H, SEQ ID NO: 43) and of the light chain of the 3A6 mAb (V-L, SEQ ID NO: 44) cDNas are determined on an ABI sequencer. Altogether ten clones of mAb3A6 light chain cDNAs from two independent experiments (RNA → cDNA →9RT-PCR) were sequenced and aligned. The subsequent amino acid sequence of V-H and V-L are shown in SEQ ID NO: 2 (V-H) and SEQ ID NO: 3 (V-L). Primers used for PCR amplification of the V_{H} and V_{L} cDNAs; all primers are synthesized by MWG Biotech, Germany.

**Table2:**

| **Primer** | **Sequence** | **SEQ ID NO:** |
|---|---|---|
| 5'-V_{L} leader | gctatggccATCGAAGCCCCAGCTCAG | 39 |
| 3'-Cκ | ttaggaattcCTAACACTCTCCCCTGTTGAAG | 40 |
| 5'-V_{H} leader | aatgtcgaccATGGAGTTTGGGCTGAGCTGGG | 41 |
| 3'-C_{H} hinge | ttagTTATGGGCACGGTGGGCATGTGTGAG | 42 |

### Cloning of the IgG4 expression vectors

### Molecular cloning of the V_{H} region

The V_{H} cDNA is amplified by PCR from the recombinant pCRII-plasmid using the primers #1 and #2. The resulting PCR-fragment is cut with *Bst*Ell and subcloned into the *Hinc*II*lBst*EII site of HCcassAAL generating the intermediate plasmid nogohccass. By using the primer IgG4HC5' an amino acid exchange (glutamine instead of aspartic acid) in the heavy chain leader in position -2 is achieved. The correct sequence is verified by automated sequencing and the fragment V_{H} cDNA is released by *Xba*I/*Bam*HI digest. Ligation into *Bam*HI/*Xba*I digested hcMCPfin resulted in the final AnogoHC3A6 expression construct.

### Molecular cloning of the V_{L} region

The V_{L} cDNA is amplified by PCR from the recombinant pCRII-plasmid using the primers #3 and #4 thereby introducing a Mlul and a Hindlll site. By introducing the Hindlll restriction site an amino acid exchange (R→K) in the joining region takes place. This results in changing the J5 type joining region into a J2 type. The resulting PCR-fragment is subcloned into pCRII-blunt and the sequence is verified. The correct fragment is released by Mlul/Hindlll digest and ligated into the expression vector LCvec-AAL160 thereby creating the final plasmid AnogoLC3A6.

| Primer # | Description | Sequence | SEQ ID NO: |
|---|---|---|---|
| 1 | IgG4HC5' | CAGGCAGAGGTGCAGCTGGTGGAGTCTGG | |
| 2 | IgG4HC3' | aaaTTGGTGGAGGCTGAGGAGACG | |
| 3 | IgG4LC5' | aaaaacgcgttgtGAAATTGTGTTGACACAGTCT | |
| 4 | IgG4LC3' | aaaaaagcttTGTCCCTTGGCCGAAGGTGATC | |

### Characterization of the human 3A6 mAb

### Example 6: Binding of 3A6 and Fab to human Nogo-A using ELISA

Greiner 96 well PS plates (#655161) are coated with 0.4-2µg/ml Nogo protein fragments in PBS (100µl/well) covered and incubated 4 hours at room temperature. Plates are flicked and refilled with 200ul/well blocking buffer (PBS+2% BSA), covered and incubated. 1h at RT or overnight at 4 °C, then washed 3 times with water and 1 time with PBS. Different concentrations of human 3A6 IgG1 , IgG4 mAb or 3A6 Fab are diluted in PBS +2% BSA (100 µl/well), and incubated 2h at RT or overnight at 4 °C. Wash step is repeated and Goat anti-human IgG conjugated with horse radish peroxidase (HRP) at a dilution of 1:5000 (Jackson Immuno Research #109-036-098) or Donkey anti-human HRP at a dilution of 1:5000 (Jackson Immuno Research 709-035-149) for 3A6Fab in PBS/0.1 %BSA /0.1 %Nonidet 40 (100 µl/well) is added and incubated. 2h at RT or overnight at 4 °C and wash step is repeated. HRP reaction is started by adding 100 µl/well BM blue POD (Roche #1484281) and incubated in the dark at RT for 15 minutes . H2SO4 50µl/well 1M is added to stop HRP substrate reaction and the optical density is determinated using a microplate reader (Packard Spectra Count) set to 450nm.

### Results:

The human 3A6 IgG1, IgG4 mAbs and 3A6 Fab binds to human NiG at very low concentrations over the range 0.01-10nM

### Example 7: Biosensor affinity measurements for mouse 3A6-IgG1, 3A6-IgG4 and 3A6 Fab to Nogo-A domains

The affinity of the mouse 3A6-IgG1 mAb, 3A6-IgG4 mAb, and of the 3A6 Fab are measured by surface plasmon resonance (SPR) using a BIAcore 2000 optical biosensor (Biacore, Uppsala, Sweden) according to the manufacture's instructions. Recombinant human NIG is covalently immobilized on a flow cell of a CM5 sensor chip using amine-coupling chemistry. Briefly; the carboxymethlyladed dextran matrix is activated by injecting 35µl of a solution containing 0.025M NHS and 0.1 M EDC. For the immobilization on the sensor chip the recombinant human NIG is diluted in 0.01 M citrate buffer at pH 4 and injected at a flow rate of 5µl/min to achieve coupling levels allowing affinity measurements. The deactivation of the remaining NHS-ester group is performed by injection of 35µl of 1 M ethanolamine hydrochloride (pH 8.5). The surface of the sensor chip is regenerated by injecting 5µl 0.1M HCl. For the measurement of the affinity the antibodies are injected at different concentrations, ranging from 0.50nM to 100nM at a flow rate of 200 µl/min. After each injection the sensor chip surface is regenerated with the injection of 10 µl 0.1 M HCl without loss of binding activity on the surface. The kinetic constants, ka and kd and the affinity constants KA and KD are evaluated using the BIAevaluations 3.0 software supplied by the manufacturer.
*Affinity measurement in BIAcore:* The kinietc and the affinity binding constants of the mouse 3A6-IgGl mAb, 3A6-IgG4 mAb, and of the 3A6 derived monovalent Fab fragment to recombinat human NogoA are measured in real time using surface plasmon resonance (SPR) technology (Biacore). For this analysis recombinant human NIG is coupled on a sensor chip surface and different concentrations of the antibodies are injected. Kinetic parameters of the binding interactions are derived from the sensorgrams by non-linear curve fitting. The affinity constants at equilibrium to human NIG for the antibodies were in the range of KDs 0.14nM to 2.7nM for 3A6-IgG4, 3A6-IgG1, 3A6 Fab

### Example 8: 3A6 mAb Epitope identification with Pepspot Analysis:

Pepspot membrane is purchased from Jerini Peptide Technologies , Berlin, Germany. Before the first incubation the membrane is rinsed with ethanol for 1 minute and three times with TBS for 10 minutes. Before each incubation with the first antibody the membrane is incubated in blocking buffer over night at 4°C. After washing for 10 minutes with TBS-T the membrane is incubated with the first antibody in blocking buffer for 3 hours at RT. Antibody concentrations are c(3A6)= 0.6nM. After three washes with TBS-T for 10 minutes the membrane is incubated for 2 hours at RT with the corresponding second HRP-labeled antibody (goat-anti human IgG Fab2 from Jackson immune Research) at 1:500 000 dilution in blocking buffer. After three washes with TBS-T the membrane is incubated with the chemiluminescence detection reagent (ECL *Advance,* Amersham Biosciences) according to the manufacturer's instructions and exposed to film.

### Western Blot analysis with human and monkey Nogo-A proteins:

The Western Blot Analysis is carried out according to standard methods. 10 ng human NiG purified from E.coli is applied to each lane, SDS-PAGE is performed and transferred to nitrocellulose membrane. Blocking is at 4°C in blocking buffer over night. After incubation of the antibody ( 1nM in 0.5% blocking buffer) with the peptide (10-, 100- and 1000-fold molar excess of peptide, Human NiG peptide epitope
HNQQELPTALTKLVKED, Scrambled peptide H-ETQLAKLPVDLKTQE: Jerini Peptide Technologies, Berlin, Germany ) for 1 hour at RT the membrane is added to this solution and incubated 1 hour at RT on a shaker. After three washes with TBS-T for 10 minutes the membrane is incubated for 1 hour at RT with the corresponding second HRP-labeled antibody (goat-anti human IgG Fab2 from Jackson immune Research) at 1:100 000 dilution in blocking buffer. After three washes with TBS-T the membrane is incubated with the chemiluminescence detection reagent (ECL *Advance,* Amersham Biosciences) according to the manufacturer's instructions and exposed to film for 15 seconds.
The Western Blot Analysis to the cynomolgus NiG is carried out according to standard methods. Aliquots of E. coli pET28-monkey NiG cell lysates expressing monkey NiG upon induction with IPTG are applied to each lane. As a negative control cell lysates of the same cells without induction of expression is loaded. SDS-PAGE is performed and transferred to nitrocellulose membrane. Blocking is at 4°C in blocking buffer over night. After incubation of the antibody (1nM in 0.5% blocking buffer, Roche Applied Science) with the peptide (10-, 100- and 1000-fold molar excess of peptide, sequence: NQQELPIALTKLVKEED, Jerini Peptide Technologies) for 1 hour at RT the membrane is added to this solution and incubated one more hour at RT on a shaker. After three washes with TBS-T for 10 minutes the membrane is incubated for 1 hour at RT with an anti human HRP-labeled secondary antibody at 1:100 000 dilution in blocking buffer. After three washes with TBS-T the membrane is incubated with the chemiluminescence detection reagent (ECL *Advance,* Amersham Biosciences) according to the manufacturer's instructions and exposed to film for 15 seconds.

### Binding of 3A6 mAb to human and monkey peptide epitopes in ELISA

Greiner 96 well PS plates are coated with 8µg/ml scrambled peptide H-ETQLAKLPVDLKTQE, human NiG peptide epitope 3A61gG4 H-NQQELPTALTKLVKED and peptide epitope 3A6 IgG4 monkey NiG, H-NQQELPIALTKLVKEED in PBS (100µl/well) covered and incubated 4 hours at room temperature. Plates are flicked and refilled with 200µl /well blocking buffer(PBS+5% BSA), covered and incubated 1h at RT or overnight at 4 degrees centigrade , then washed 4 times with tap water, refilled with PBS and flicked.
mAb 3A6IgG4 is diluted in PBS+2%BSA (100µl/well), and incubated 2h at RT or overnight at 4 degrees. Dilutions of mAb: 10nM to 0.001nM..Wash step is repeated. 2nd.Ab is diluted in PBS/0.1 %BSA /0.1 %Nonidet 40 (100 µl/well) and incubated 2h at RT or overnight at 4 degrees. Wash step is repeated. 100µl/well BM blue POD substrate are added and incubated in the dark at room temperature 15 minutes and 50µl/well 1 M H2SO4 is added to stop HRP substrate reaction. The OD is determined using a microplate reader set to 450nm.

### Results:

**Epitope mapping of the human 3A6 mAb:** The epitope mapping results in a sequence ELPTALTKLV in human NiG protein.

### Competition of mAb 3A6 binding to human NiG in western blot with synthetic peptide:

To confirm the result obtained by the pepspot technique western blot competition experiment is performed. A synthetic 16-mer containing the putative epitope sequence (NQQELPTALTKLVKED) is used to compete with full length human NiG for binding to the 3A6 antibody. Prior to incubation with membrane bound human NiG the3A6 antibody (1 nM) is incubated for 1 hour with the synthetic peptide using different molar ratios of peptide to antibody. A 10-fold molar excess of peptide shows a significant decrease in the detected signal for human NiG (produced in E. coli). A 100-fold excess results in a further decrease of the signal, and a 1000-fold molar excess of the peptide nearly completely inhibits the binding of the 3A6 to human NiG. In contrast a 1000-fold excess of a peptide with the same amino acid content but with a different sequence (scrambled) does not have any effect on the binding of the antibody to human NiG.

### mAb 3A6 binding to monkey NiG : competition with synthetic peptide epitope

A synthetic 17-mer containing the epitope sequence (NQQELPIALTKLVKEED) is used to compete with full length cynomolgus monkey NiG expressed in E. coli for binding to the 3A6 antibody. Prior to incubation with membrane bound monkey NiG the3A6 antibody (1 nM) is incubated for 1 hour with the synthetic peptide using different molar ratios of peptide to antibody. A 100-fold excess results in a decrease of the signal, and a 1000-fold molar excess of the peptide substantially inhibits the binding of the 3A6 to monkey NiG. In contrast a 1000-fold excess of a peptide with the same amino acid content but with a different sequence (scrambled) does not have any effect on the binding of the antibody to human NiG.

### Binding of 3A6 IgG4 to the human and monkey NiG peptide epitope in ELISA

Detailed binding analyses of the mAb to the epitope and scrambled sequence are performed using ELISA . The results show clearly that the mAb binds in a concentration dependent manner at very low concentrations (0.001 to 1.0 nM) to monkey and human peptide epitopes comparable to its KD in BIAcore for human NiG of 0.14 nM. Moreover binding is specific with no binding to the scrambled control peptide.

## Claims

1. A human or chimeric or humanized monoclonal antibody, or a F(ab')₂ or Fab fragment of such an antibody, which binds the human NogoA_342-357 epitope defined by amino acid sequence NQQELPTALTKLVKED.

2. The antibody of claim 1, which binds said human NogoA epitope with a dissociation constant (Kd) < 10 nM.

3. The antibody according to claims 1 or 2, in which the constant part or fragment thereof of the human heavy chain is of the y4-type and the constant part or fragment thereof of the human light chain is of the κ-type.

4. The use of an antibody as defined in claims 1 to 3 as a pharmaceutical.

5. The use of an antibody as defined in claims 1 to 3 for the manufacture of a medicament for the treatment of nerve repair or of a disease of the peripheral or central nervous system.

6. A pharmaceutical composition comprising an antibody or a fragment thereof as defined in claims 1-3 in association with at least one pharmaceutically acceptable carrier or diluent.

7. An epitope of the human NogoA protein consisting of the amino acid sequence of human NogoA_342-357 or consisting of amino acid sequence NQQELPTALTKLVKED.

8. The antibody or a fragment thereof according to any one of claims 1-3 for treating disorders associated with nerve repair.

9. The antibody or a fragment thereof according to any one of claims 1-3 for treating disorders of the peripheral or central nervous system.


SEQUENCE LISTING

<110>  Novartis AG

<120>  Organic Compound

<130>  4-32761P1/UNZ

<160>  44

<170>  PatentIn version 3.1

<210>  1

<211>  18

<212>  PRT

<213>  Rattus norvegicus

<220>

<221>  PEPTIDE

<222>  (1)..(18)

<223>  rat NogoA_342-357

<400>  1

Ser Tyr Asp Ser Ile Lys Leu Glu Pro Glu Asn Pro Pro Pro Tyr Glu
1               5                   10                  15

Glu Ala

<210>  2

<211>  221

<212>  PRT

<213>  Mus musculus

<220>

<221>  CHAIN

<222>  (1)..(221)

<223>  Variable part of Heavy Chain of 3A6 with leader sequence

<400>  2

Met Asp Phe Gly Leu Ile Phe Phe Ile Val Gly Leu Leu Lys Gly Val
1               5                   10                  15

Gln Cys Glu Val Lys Leu Leu Glu Ser Gly Gly Gly Leu Val Gln Pro
            20                  25                  30

Gly Gly Ser Leu Lys Leu Ser Cys Val Val Ser Gly Phe Asp Phe Arg
        35                  40                  45

Arg Asn Trp Met Ser Trp Val Arg Gln Ala Pro Gly Lys Gly Leu Glu
    50                  55                  60

Trp Ile Gly Glu Ile Asn Pro Asp Ser Ser Lys Ile Asn Tyr Thr Pro
65                  70                  75                  80

Ser Leu Lys Asp Lys Phe Ile Ile Ser Arg Asp Asn Ala Lys Asn Thr

85                  90                  95

Leu Tyr Leu Gln Val Ser Thr Val Arg Ser Glu Asp Thr Ala Leu Tyr
            100                 105                 110

Tyr Cys Val Arg Pro Val Trp Met Tyr Ala Met Asp Tyr Trp Gly Gln
        115                 120                 125

Gly Thr Ser Val Thr Val Ser Ser Ala Lys Thr Thr Pro Pro Ser Val
    130                 135                 140

Tyr Pro Leu Ala Pro Gly Ser Ala Ala Gln Thr Asn Ser Met Val Thr
145                 150                 155                 160

Leu Gly Cys Leu Val Lys Gly Tyr Phe Pro Glu Pro Val Thr Val Thr
                165                 170                 175

Trp Asn Ser Gly Ser Leu Ser Ser Gly Val His Thr Phe Pro Ala Val
            180                 185                 190

Leu Gln Ser Asp Leu Tyr Thr Leu Ser Ser Ser Val Thr Val Pro Ser
        195                 200                 205

Ser Thr Trp Pro Ser Glu Thr Val Thr Cys Asn Val Ala
    210                 215                 220

<210>  3

<211>  238

<212>  PRT

<213>  Mus musculus

<220>

<221>  CHAIN

<222>  (1)..(238)

<223>  Light Chain of 3A6 with leader sequence

<400>  3

Met Ser Pro Ala Gln Phe Leu Phe Leu Leu Val Leu Trp Ile Arg Glu
1               5                   10                  15

Thr Ser Gly Asp Val Leu Leu Thr Gln Thr Pro Leu Thr Leu Ser Ile
            20                  25                  30

Thr Ile Gly Gln Pro Ala Ser Ile Ser Cys Lys Ser Ser Gln Ser Leu

35                  40                  45

Leu His Ser Asp Gly Lys Thr Tyr Leu Asn Trp Leu Leu Gln Arg Pro
    50                  55                  60

Gly Gln Ser Pro Lys Arg Leu Ile Tyr Leu Val Ser Lys Leu Asp Ser
65                  70                  75                  80

Gly Val Pro Asp Arg Phe Thr Gly Ser Gly Ser Gly Thr Asp Phe Thr
                85                  90                  95

Leu Lys Ile Ser Arg Val Glu Ala Glu Asp Leu Gly Leu Tyr Tyr Cys
            100                 105                 110

Trp Gln Gly Thr His Phe Pro Gln Thr Phe Gly Gly Gly Thr Lys Leu
        115                 120                 125

Glu Ile Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Gly Glu Cys
225                 230                 235

<210>  4

<211>  3919

<212>  DNA

<213>  Homo sapiens

<220>

<221>  CDS

<222>  (1)..(3579)

<223>  Human NogoA

<400>  4
atg gaa gac ctg gac cag tct cct ctg gtc tcg tcc tcg gac agc cca        48
Met Glu Asp Leu Asp Gln Ser Pro Leu Val Ser Ser Ser Asp Ser Pro
1               5                   10                  15

ccc cgg ccg cag ccc gcg ttc aag tac cag ttc gtg agg gag ccc gag        96
Pro Arg Pro Gln Pro Ala Phe Lys Tyr Gln Phe Val Arg Glu Pro Glu
            20                  25                  30

gac gag gag gaa gaa gag gag gag gaa gag gag gac gag gac gaa gac       144
Asp Glu Glu Glu Glu Glu Glu Glu Glu Glu Glu Asp Glu Asp Glu Asp
        35                  40                  45

ctg gag gag ctg gag gtg ctg gag agg aag ccc gcc gcc ggg ctg tcc       192
Leu Glu Glu Leu Glu Val Leu Glu Arg Lys Pro Ala Ala Gly Leu Ser
    50                  55                  60

gcg gcc cca gtg ccc acc gcc cct gcc gcc ggc gcg ccc ctg atg gac       240
Ala Ala Pro Val Pro Thr Ala Pro Ala Ala Gly Ala Pro Leu Met Asp
65                  70                  75                  80

ttc gga aat gac ttc gtg ccg ccg gcg ccc cgg gga ccc ctg ccg gcc       288
Phe Gly Asn Asp Phe Val Pro Pro Ala Pro Arg Gly Pro Leu Pro Ala
                85                  90                  95

gct ccc ccc gtc gcc ccg gag cgg cag ccg tct tgg gac ccg agc ccg       336
Ala Pro Pro Val Ala Pro Glu Arg Gln Pro Ser Trp Asp Pro Ser Pro

            100                 105                 110

gtg tcg tcg acc gtg ccc gcg cca tcc ccg ctg tct gct gcc gca gtc 384
Val Ser Ser Thr Val Pro Ala Pro Ser Pro Leu Ser Ala Ala Ala Val
        115                 120                 125

tcg ccc tcc aag ctc cct gag gac gac gag cct ccg gcc cgg cct ccc 432
Ser Pro Ser Lys Leu Pro Glu Asp Asp Glu Pro Pro Ala Arg Pro Pro
    130                 135                 140

cct cct ccc ccg gcc agc gtg agc ccc cag gca gag ccc gtg tgg acc 480
Pro Pro Pro Pro Ala Ser Val Ser Pro Gln Ala Glu Pro Val Trp Thr
145                 150                 155                 160

ccg cca gcc ccg gct ccc gcc gcg ccc ccc tcc acc ccg gcc gcg ccc 528
Pro Pro Ala Pro Ala Pro Ala Ala Pro Pro Ser Thr Pro Ala Ala Pro
                165                 170                 175

aag cgc agg ggc tcc tcg ggc tca gtg gat gag acc ctt ttt gct ctt 576
Lys Arg Arg Gly Ser Ser Gly Ser Val Asp Glu Thr Leu Phe Ala Leu
            180                 185                 190

cct gct gca tct gag cct gtg ata cgc tcc tct gca gaa aat atg gac 624
Pro Ala Ala Ser Glu Pro Val Ile Arg Ser Ser Ala Glu Asn Met Asp
        195                 200                 205

ttg aag gag cag cca ggt aac act att tcg gct ggt caa gag gat ttc 672
Leu Lys Glu Gln Pro Gly Asn Thr Ile Ser Ala Gly Gln Glu Asp Phe
    210                 215                 220

cca tct gtc ctg ctt gaa act gct gct tct ctt cct tct ctg tct cct 720
Pro Ser Val Leu Leu Glu Thr Ala Ala Ser Leu Pro Ser Leu Ser Pro
225                 230                 235                 240

ctc tca gcc gct tct ttc aaa gaa cat gaa tac ctt ggt aat ttg tca 768
Leu Ser Ala Ala Ser Phe Lys Glu His Glu Tyr Leu Gly Asn Leu Ser
                245                 250                 255

aca gta tta ccc act gaa gga aca ctt caa gaa aat gtc agt gaa gct 816
Thr Val Leu Pro Thr Glu Gly Thr Leu Gln Glu Asn Val Ser Glu Ala
            260                 265                 270

tct aaa gag gtc tca gag aag gca aaa act cta ctc ata gat aga gat 864
Ser Lys Glu Val Ser Glu Lys Ala Lys Thr Leu Leu Ile Asp Arg Asp
        275                 280                 285

tta aca gag ttt tca gaa tta gaa tac tca gaa atg gga tca tcg ttc 912
Leu Thr Glu Phe Ser Glu Leu Glu Tyr Ser Glu Met Gly Ser Ser Phe
    290                 295                 300

agt gtc tct cca aaa gca gaa tct gcc gta ata gta gca aat cct agg 960
Ser Val Ser Pro Lys Ala Glu Ser Ala Val Ile Val Ala Asn Pro Arg
305                 310                 315                 320

gaa gaa ata atc gtg aaa aat aaa gat gaa gaa gag aag tta gtt agt 1008
Glu Glu Ile Ile Val Lys Asn Lys Asp Glu Glu Glu Lys Leu Val Ser
                325                 330                 335

aat aac atc ctt cat aat caa caa gag tta cct aca gct ctt act aaa 1056
Asn Asn Ile Leu His Asn Gln Gln Glu Leu Pro Thr Ala Leu Thr Lys
            340                 345                 350

ttg gtt aaa gag gat gaa gtt gtg tct tca gaa aaa gca aaa gac agt 1104
Leu Val Lys Glu Asp Glu Val Val Ser Ser Glu Lys Ala Lys Asp Ser
        355                 360                 365

ttt aat gaa aag aga gtt gca gtg gaa gct cct atg agg gag gaa tat 1152
Phe Asn Glu Lys Arg Val Ala Val Glu Ala Pro Met Arg Glu Glu Tyr
    370                 375                 380

gca gac ttc aaa cca ttt gag cga gta tgg gaa gtg aaa gat agt aag 1200
Ala Asp Phe Lys Pro Phe Glu Arg Val Trp Glu Val Lys Asp Ser Lys
385                 390                 395                 400

gaa gat agt gat atg ttg gct gct gga ggt aaa atc gag agc aac ttg 1248
Glu Asp Ser Asp Met Leu Ala Ala Gly Gly Lys Ile Glu Ser Asn Leu
                405                 410                 415

gaa agt aaa gtg gat aaa aaa tgt ttt gca gat agc ctt gag caa act 1296
Glu Ser Lys Val Asp Lys Lys Cys Phe Ala Asp Ser Leu Glu Gln Thr
            420                 425                 430

aat cac gaa aaa gat agt gag agt agt aat gat gat act tct ttc ccc 1344
Asn His Glu Lys Asp Ser Glu Ser Ser Asn Asp Asp Thr Ser Phe Pro
        435                 440                 445

agt acg cca gaa ggt ata aag gat cgt tca gga gca tat atc aca tgt 1392
Ser Thr Pro Glu Gly Ile Lys Asp Arg Ser Gly Ala Tyr Ile Thr Cys
    450                 455                 460

gct ccc ttt aac cca gca gca act gag agc att gca aca aac att ttt 1440
Ala Pro Phe Asn Pro Ala Ala Thr Glu Ser Ile Ala Thr Asn Ile Phe
465                 470                 475                 480

cct ttg tta gga gat cct act tca gaa aat aag acc gat gaa aaa aaa 1488
Pro Leu Leu Gly Asp Pro Thr Ser Glu Asn Lys Thr Asp Glu Lys Lys
                485                 490                 495

ata gaa gaa aag aag gcc caa ata gta aca gag aag aat act agc acc 1536

Ile Glu Glu Lys Lys Ala Gln Ile Val Thr Glu Lys Asn Thr Ser Thr
            500                 505                 510

aaa aca tca aac cct ttt ctt gta gca gca cag gat tct gag aca gat 1584
Lys Thr Ser Asn Pro Phe Leu Val Ala Ala Gln Asp Ser Glu Thr Asp
        515                 520                 525

tat gtc aca aca gat aat tta aca aag gtg act gag gaa gtc gtg gca 1632
Tyr Val Thr Thr Asp Asn Leu Thr Lys Val Thr Glu Glu Val Val Ala
    530                 535                 540

aac atg cct gaa ggc ctg act cca gat tta gta cag gaa gca tgt gaa 1680
Asn Met Pro Glu Gly Leu Thr Pro Asp Leu Val Gln Glu Ala Cys Glu
545                 550                 555                 560

agt gaa ttg aat gaa gtt act ggt aca aag att gct tat gaa aca aaa 1728
Ser Glu Leu Asn Glu Val Thr Gly Thr Lys Ile Ala Tyr Glu Thr Lys
                565                 570                 575

atg gac ttg gtt caa aca tca gaa gtt atg caa gag tca ctc tat cct 1776
Met Asp Leu Val Gln Thr Ser Glu Val Met Gln Glu Ser Leu Tyr Pro
            580                 585                 590

gca gca cag ctt tgc cca tca ttt gaa gag tca gaa gct act cct tca 1824
Ala Ala Gln Leu Cys Pro Ser Phe Glu Glu Ser Glu Ala Thr Pro Ser
        595                 600                 605

cca gtt ttg cct gac att gtt atg gaa gca cca ttg aat tct gca gtt 1872
Pro Val Leu Pro Asp Ile Val Met Glu Ala Pro Leu Asn Ser Ala Val
    610                 615                 620

cct agt gct ggt gct tcc gtg ata cag ccc agc tca tca cca tta gaa 1920
Pro Ser Ala Gly Ala Ser Val Ile Gln Pro Ser Ser Ser Pro Leu Glu

625                 630                 635                 640

gct tct tca gtt aat tat gaa agc ata aaa cat gag cct gaa aac ccc      1968
Ala Ser Ser Val Asn Tyr Glu Ser Ile Lys His Glu Pro Glu Asn Pro
                645                 650                 655

cca cca tat gaa gag gcc atg agt gta tca cta aaa aaa gta tca gga      2016
Pro Pro Tyr Glu Glu Ala Met Ser Val Ser Leu Lys Lys Val Ser Gly
            660                 665                 670

ata aag gaa gaa att aaa gag cct gaa aat att aat gca gct ctt caa      2064
Ile Lys Glu Glu Ile Lys Glu Pro Glu Asn Ile Asn Ala Ala Leu Gln
        675                 680                 685

gaa aca gaa gct cct tat ata tct att gca tgt gat tta att aaa gaa      2112
Glu Thr Glu Ala Pro Tyr Ile Ser Ile Ala Cys Asp Leu Ile Lys Glu
    690                 695                 700

aca aag ctt tct gct gaa cca gct ccg gat ttc tct gat tat tca gaa      2160
Thr Lys Leu Ser Ala Glu Pro Ala Pro Asp Phe Ser Asp Tyr Ser Glu
705                 710                 715                 720

atg gca aaa gtt gaa cag cca gtg cct gat cat tct gag cta gtt gaa      2208
Met Ala Lys Val Glu Gln Pro Val Pro Asp His Ser Glu Leu Val Glu
                725                 730                 735

gat tcc tca cct gat tct gaa cca gtt gac tta ttt agt gat gat tca      2256
Asp Ser Ser Pro Asp Ser Glu Pro Val Asp Leu Phe Ser Asp Asp Ser
            740                 745                 750

ata cct gac gtt cca caa aaa caa gat gaa act gtg atg ctt gtg aaa      2304
Ile Pro Asp Val Pro Gln Lys Gln Asp Glu Thr Val Met Leu Val Lys
        755                 760                 765

gaa agt ctc act gag act tca ttt gag tca atg ata gaa tat gaa aat 2352
Glu Ser Leu Thr Glu Thr Ser Phe Glu Ser Met Ile Glu Tyr Glu Asn
    770                 775                 780

aag gaa aaa ctc agt gct ttg cca cct gag gga gga aag cca tat ttg 2400
Lys Glu Lys Leu Ser Ala Leu Pro Pro Glu Gly Gly Lys Pro Tyr Leu
785                 790                 795                 800

gaa tct ttt aag ctc agt tta gat aac aca aaa gat acc ctg tta cct 2448
Glu Ser Phe Lys Leu Ser Leu Asp Asn Thr Lys Asp Thr Leu Leu Pro
                805                 810                 815

gat gaa gtt tca aca ttg agc aaa aag gag aaa att cct ttg cag atg 2496
Asp Glu Val Ser Thr Leu Ser Lys Lys Glu Lys Ile Pro Leu Gln Met
            820                 825                 830

gag gag ctc agt act gca gtt tat tca aat gat gac tta ttt att tct 2544
Glu Glu Leu Ser Thr Ala Val Tyr Ser Asn Asp Asp Leu Phe Ile Ser
        835                 840                 845

aag gaa gca cag ata aga gaa act gaa acg ttt tca gat tca tct cca 2592
Lys Glu Ala Gln Ile Arg Glu Thr Glu Thr Phe Ser Asp Ser Ser Pro
    850                 855                 860

att gaa att ata gat gag ttc cct aca ttg atc agt tct aaa act gat 2640
Ile Glu Ile Ile Asp Glu Phe Pro Thr Leu Ile Ser Ser Lys Thr Asp
865                 870                 875                 880

tca ttt tct aaa tta gcc agg gaa tat act gac cta gaa gta tcc cac 2688
Ser Phe Ser Lys Leu Ala Arg Glu Tyr Thr Asp Leu Glu Val Ser His
                885                 890                 895

aaa agt gaa att gct aat gcc ccg gat gga gct ggg tca ttg cct tgc     2736
Lys Ser Glu Ile Ala Asn Ala Pro Asp Gly Ala Gly Ser Leu Pro Cys
            900                 905                 910

aca gaa ttg ccc cat gac ctt tct ttg aag aac ata caa ccc aaa gtt     2784
Thr Glu Leu Pro His Asp Leu Ser Leu Lys Asn Ile Gln Pro Lys Val
        915                 920                 925

gaa gag aaa atc agt ttc tca gat gac ttt tct aaa aat ggg tct gct     2832
Glu Glu Lys Ile Ser Phe Ser Asp Asp Phe Ser Lys Asn Gly Ser Ala
    930                 935                 940

aca tca aag gtg ctc tta ttg cct cca gat gtt tct gct ttg gcc act     2880
Thr Ser Lys Val Leu Leu Leu Pro Pro Asp Val Ser Ala Leu Ala Thr
945                 950                 955                 960

caa gca gag ata gag agc ata gtt aaa ccc aaa gtt ctt gtg aaa gaa     2928
Gln Ala Glu Ile Glu Ser Ile Val Lys Pro Lys Val Leu Val Lys Glu
                965                 970                 975

gct gag aaa aaa ctt cct tcc gat aca gaa aaa gag gac aga tca cca     2976
Ala Glu Lys Lys Leu Pro Ser Asp Thr Glu Lys Glu Asp Arg Ser Pro
            980                 985                 990

tct gct ata ttt tca gca gag ctg  agt aaa act tca gtt  gtt gac ctc     3024
Ser Ala Ile Phe Ser Ala Glu Leu  Ser Lys Thr Ser Val  Val Asp Leu
        995                 1000                1005

ctg tac  tgg aga gac att aag  aag act gga gtg gtg  ttt ggt gcc     3069
Leu Tyr  Trp Arg Asp Ile Lys  Lys Thr Gly Val Val  Phe Gly Ala
    1010                1015                1020

agc cta  ttc ctg ctg ctt tca  ttg aca gta ttc agc  att gtg agc     3114

Ser Leu Phe Leu Leu Leu Ser Leu Thr Val Phe Ser Ile Val Ser
    1025                1030                1035

gta aca gcc tac att gcc ttg gcc ctg ctc tct gtg acc atc agc 3159
Val Thr Ala Tyr Ile Ala Leu Ala Leu Leu Ser Val Thr Ile Ser
    1040                1045                1050

ttt agg ata tac aag ggt gtg atc caa gct atc cag aaa tca gat 3204
Phe Arg Ile Tyr Lys Gly Val Ile Gln Ala Ile Gln Lys Ser Asp
    1055                1060                1065

gaa ggc cac cca ttc agg gca tat ctg gaa tct gaa gtt gct ata 3249
Glu Gly His Pro Phe Arg Ala Tyr Leu Glu Ser Glu Val Ala Ile
    1070                1075                1080

tct gag gag ttg gtt cag aag tac agt aat tct gct ctt ggt cat 3294
Ser Glu Glu Leu Val Gln Lys Tyr Ser Asn Ser Ala Leu Gly His
    1085                1090                1095

gtg aac tgc acg ata aag gaa ctc agg cgc ctc ttc tta gtt gat 3339
Val Asn Cys Thr Ile Lys Glu Leu Arg Arg Leu Phe Leu Val Asp
    1100                1105                1110

gat tta gtt gat tct ctg aag ttt gca gtg ttg atg tgg gta ttt 3384
Asp Leu Val Asp Ser Leu Lys Phe Ala Val Leu Met Trp Val Phe
    1115                1120                1125

acc tat gtt ggt gcc ttg ttt aat ggt ctg aca cta ctg att ttg 3429
Thr Tyr Val Gly Ala Leu Phe Asn Gly Leu Thr Leu Leu Ile Leu
    1130                1135                1140

gct ctc att tca ctc ttc agt gtt cct gtt att tat gaa cgg cat 3474
Ala Leu Ile Ser Leu Phe Ser Val Pro Val Ile Tyr Glu Arg His

1145                1150                1155

cag gca  cag ata gat cat tat  cta gga ctt gca aat  aag aat gtt      3519
Gln Ala  Gln Ile Asp His Tyr  Leu Gly Leu Ala Asn  Lys Asn Val
    1160                1165                1170

aaa gat  gct atg gct aaa atc  caa gca aaa atc cct  gga ttg aag      3564
Lys Asp  Ala Met Ala Lys Ile  Gln Ala Lys Ile Pro  Gly Leu Lys
    1175                1180                1185

cgc aaa  gct gaa tga aaacgcccaa aataattagt aggagttcat ctttaaaggg    3619
Arg Lys  Ala Glu
    1190

gatattcatt tgattatacg ggggagggtc agggaagaac gaaccttgac gttgcagtgc   3679

agtttcacag atcgttgtta gatctttatt tttagccatg cactgttgtg aggaaaaatt   3739

acctgtcttg actgccatgt gttcatcatc ttaagtattg taagctgcta tgtatggatt   3799

taaaccgtaa tcatatcttt ttcctatctg aggcactggt ggaataaaaa acctgtatat   3859

tttactttgt tgcagatagt cttgccgcat cttggcaagt tgcagagatg gtggagctag   3919

<210>  5

<211>  1192

<212>  PRT

<213>  Homo sapiens

<400>  5

Met Glu Asp Leu Asp Gln Ser Pro Leu Val Ser Ser Ser Asp Ser Pro
1               5                   10                  15

Pro Arg Pro Gln Pro Ala Phe Lys Tyr Gln Phe Val Arg Glu Pro Glu
            20                  25                  30

Asp Glu Glu Glu Glu Glu Glu Glu Glu Glu Glu Asp Glu Asp Glu Asp
        35                  40                  45

Leu Glu Glu Leu Glu Val Leu Glu Arg Lys Pro Ala Ala Gly Leu Ser
    50                  55                  60

Ala Ala Pro Val Pro Thr Ala Pro Ala Ala Gly Ala Pro Leu Met Asp
65                  70                  75                  80

Phe Gly Asn Asp Phe Val Pro Pro Ala Pro Arg Gly Pro Leu Pro Ala
                85                  90                  95

Ala Pro Pro Val Ala Pro Glu Arg Gln Pro Ser Trp Asp Pro Ser Pro
            100                 105                 110

Val Ser Ser Thr Val Pro Ala Pro Ser Pro Leu Ser Ala Ala Ala Val

        115                 120                 125

Ser Pro Ser Lys Leu Pro Glu Asp Asp Glu Pro Pro Ala Arg Pro Pro
    130                 135                 140

Pro Pro Pro Pro Ala Ser Val Ser Pro Gln Ala Glu Pro Val Trp Thr
145                 150                 155                 160

Pro Pro Ala Pro Ala Pro Ala Ala Pro Pro Ser Thr Pro Ala Ala Pro
                165                 170                 175

Lys Arg Arg Gly Ser Ser Gly Ser Val Asp Glu Thr Leu Phe Ala Leu
            180                 185                 190

Pro Ala Ala Ser Glu Pro Val Ile Arg Ser Ser Ala Glu Asn Met Asp
        195                 200                 205

Leu Lys Glu Gln Pro Gly Asn Thr Ile Ser Ala Gly Gln Glu Asp Phe
    210                 215                 220

Pro Ser Val Leu Leu Glu Thr Ala Ala Ser Leu Pro Ser Leu Ser Pro
225                 230                 235                 240

Leu Ser Ala Ala Ser Phe Lys Glu His Glu Tyr Leu Gly Asn Leu Ser
                245                 250                 255

Thr Val Leu Pro Thr Glu Gly Thr Leu Gln Glu Asn Val Ser Glu Ala
            260                 265                 270

Ser Lys Glu Val Ser Glu Lys Ala Lys Thr Leu Leu Ile Asp Arg Asp
        275                 280                 285

Leu Thr Glu Phe Ser Glu Leu Glu Tyr Ser Glu Met Gly Ser Ser Phe
    290                 295                 300

Ser Val Ser Pro Lys Ala Glu Ser Ala Val Ile Val Ala Asn Pro Arg
305                 310                 315                 320

Glu Glu Ile Ile Val Lys Asn Lys Asp Glu Glu Glu Lys Leu Val Ser
                325                 330                 335

Asn Asn Ile Leu His Asn Gln Gln Glu Leu Pro Thr Ala Leu Thr Lys
            340                 345                 350

Leu Val Lys Glu Asp Glu Val Val Ser Ser Glu Lys Ala Lys Asp Ser
        355                 360                 365

Phe Asn Glu Lys Arg Val Ala Val Glu Ala Pro Met Arg Glu Glu Tyr
    370                 375                 380

Ala Asp Phe Lys Pro Phe Glu Arg Val Trp Glu Val Lys Asp Ser Lys
385                 390                 395                 400

Glu Asp Ser Asp Met Leu Ala Ala Gly Gly Lys Ile Glu Ser Asn Leu
                405                 410                 415

Glu Ser Lys Val Asp Lys Lys Cys Phe Ala Asp Ser Leu Glu Gln Thr
            420                 425                 430

Asn His Glu Lys Asp Ser Glu Ser Ser Asn Asp Asp Thr Ser Phe Pro
        435                 440                 445

Ser Thr Pro Glu Gly Ile Lys Asp Arg Ser Gly Ala Tyr Ile Thr Cys
    450                 455                 460

Ala Pro Phe Asn Pro Ala Ala Thr Glu Ser Ile Ala Thr Asn Ile Phe
465                 470                 475                 480

Pro Leu Leu Gly Asp Pro Thr Ser Glu Asn Lys Thr Asp Glu Lys Lys
                485                 490                 495

Ile Glu Glu Lys Lys Ala Gln Ile Val Thr Glu Lys Asn Thr Ser Thr
            500                 505                 510

Lys Thr Ser Asn Pro Phe Leu Val Ala Ala Gln Asp Ser Glu Thr Asp
        515                 520                 525

Tyr Val Thr Thr Asp Asn Leu Thr Lys Val Thr Glu Glu Val Val Ala
    530                 535                 540

Asn Met Pro Glu Gly Leu Thr Pro Asp Leu Val Gln Glu Ala Cys Glu
545                 550                 555                 560

Ser Glu Leu Asn Glu Val Thr Gly Thr Lys Ile Ala Tyr Glu Thr Lys
                565                 570                 575

Met Asp Leu Val Gln Thr Ser Glu Val Met Gln Glu Ser Leu Tyr Pro
            580                 585                 590

Ala Ala Gln Leu Cys Pro Ser Phe Glu Glu Ser Glu Ala Thr Pro Ser
        595                 600                 605

Pro Val Leu Pro Asp Ile Val Met Glu Ala Pro Leu Asn Ser Ala Val
    610                 615                 620

Pro Ser Ala Gly Ala Ser Val Ile Gln Pro Ser Ser Ser Pro Leu Glu
625                 630                 635                 640

Ala Ser Ser Val Asn Tyr Glu Ser Ile Lys His Glu Pro Glu Asn Pro

                645                 650                 655

Pro Pro Tyr Glu Glu Ala Met Ser Val Ser Leu Lys Lys Val Ser Gly
            660                 665                 670

Ile Lys Glu Glu Ile Lys Glu Pro Glu Asn Ile Asn Ala Ala Leu Gln
        675                 680                 685

Glu Thr Glu Ala Pro Tyr Ile Ser Ile Ala Cys Asp Leu Ile Lys Glu
    690                 695                 700

Thr Lys Leu Ser Ala Glu Pro Ala Pro Asp Phe Ser Asp Tyr Ser Glu
705                 710                 715                 720

Met Ala Lys Val Glu Gln Pro Val Pro Asp His Ser Glu Leu Val Glu
                725                 730                 735

Asp Ser Ser Pro Asp Ser Glu Pro Val Asp Leu Phe Ser Asp Asp Ser
            740                 745                 750

Ile Pro Asp Val Pro Gln Lys Gln Asp Glu Thr Val Met Leu Val Lys
        755                 760                 765

Glu Ser Leu Thr Glu Thr Ser Phe Glu Ser Met Ile Glu Tyr Glu Asn
    770                 775                 780

Lys Glu Lys Leu Ser Ala Leu Pro Pro Glu Gly Gly Lys Pro Tyr Leu
785                 790                 795                 800

Glu Ser Phe Lys Leu Ser Leu Asp Asn Thr Lys Asp Thr Leu Leu Pro
                805                 810                 815

Asp Glu Val Ser Thr Leu Ser Lys Lys Glu Lys Ile Pro Leu Gln Met
            820                 825                 830

Glu Glu Leu Ser Thr Ala Val Tyr Ser Asn Asp Asp Leu Phe Ile Ser
        835                 840                 845

Lys Glu Ala Gln Ile Arg Glu Thr Glu Thr Phe Ser Asp Ser Ser Pro
    850                 855                 860

Ile Glu Ile Ile Asp Glu Phe Pro Thr Leu Ile Ser Ser Lys Thr Asp
865                 870                 875                 880

Ser Phe Ser Lys Leu Ala Arg Glu Tyr Thr Asp Leu Glu Val Ser His
                885                 890                 895

Lys Ser Glu Ile Ala Asn Ala Pro Asp Gly Ala Gly Ser Leu Pro Cys
            900                 905                 910

Thr Glu Leu Pro His Asp Leu Ser Leu Lys Asn Ile Gln Pro Lys Val
        915                 920                 925

Glu Glu Lys Ile Ser Phe Ser Asp Asp Phe Ser Lys Asn Gly Ser Ala
    930                 935                 940

Thr Ser Lys Val Leu Leu Leu Pro Pro Asp Val Ser Ala Leu Ala Thr
945                 950                 955                 960

Gln Ala Glu Ile Glu Ser Ile Val Lys Pro Lys Val Leu Val Lys Glu
                965                 970                 975

Ala Glu Lys Lys Leu Pro Ser Asp Thr Glu Lys Glu Asp Arg Ser Pro
            980                 985                 990

Ser Ala Ile Phe Ser Ala Glu Leu Ser Lys Thr Ser Val Val Asp Leu
        995                 1000                1005

Leu Tyr Trp Arg Asp Ile Lys Lys Thr Gly Val Val Phe Gly Ala
    1010                1015                1020

Ser Leu Phe Leu Leu Leu Ser Leu Thr Val Phe Ser Ile Val Ser
    1025                1030                1035

Val Thr Ala Tyr Ile Ala Leu Ala Leu Leu Ser Val Thr Ile Ser
    1040                1045                1050

Phe Arg Ile Tyr Lys Gly Val Ile Gln Ala Ile Gln Lys Ser Asp
    1055                1060                1065

Glu Gly His Pro Phe Arg Ala Tyr Leu Glu Ser Glu Val Ala Ile
    1070                1075                1080

Ser Glu Glu Leu Val Gln Lys Tyr Ser Asn Ser Ala Leu Gly His
    1085                1090                1095

Val Asn Cys Thr Ile Lys Glu Leu Arg Arg Leu Phe Leu Val Asp
    1100                1105                1110

Asp Leu Val Asp Ser Leu Lys Phe Ala Val Leu Met Trp Val Phe
    1115                1120                1125

Thr Tyr Val Gly Ala Leu Phe Asn Gly Leu Thr Leu Leu Ile Leu
    1130                1135                1140

Ala Leu Ile Ser Leu Phe Ser Val Pro Val Ile Tyr Glu Arg His
    1145                1150                1155

Gln Ala Gln Ile Asp His Tyr Leu Gly Leu Ala Asn Lys Asn Val

1160                1165                1170

Lys Asp  Ala Met Ala Lys Ile  Gln Ala Lys Ile Pro  Gly Leu Lys
    1175                1180                1185

Arg Lys  Ala Glu
    1190

<210>  6

<211>  18

<212>  PRT

<213>  Homo sapiens

<220>

<221>  PEPTIDE

<222>  (1)..(18)

<223>  Human NogoA_342-357

<400>  6

Asn Tyr Glu Ser Ile Lys His Glu Pro Glu Asn Pro Pro Pro Tyr Glu
1               5                   10                  15

Glu Ala

<210>  7

<211>  819

<212>  PRT

<213>  Homo sapiens

<220>

<221>  PEPTIDE

<222>  (1)..(819)

<223>  human Nig

<400>  7

Asp Glu Thr Leu Phe Ala Leu Pro Ala Ala Ser Glu Pro Val Ile Arg
1               5                   10                  15

Ser Ser Ala Glu Asn Met Asp Leu Lys Glu Gln Pro Gly Asn Thr Ile
            20                  25                  30

Ser Ala Gly Gln Glu Asp Phe Pro Ser Val Leu Leu Glu Thr Ala Ala
        35                  40                  45

Ser Leu Pro Ser Leu Ser Pro Leu Ser Ala Ala Ser Phe Lys Glu His
    50                  55                  60

Glu Tyr Leu Gly Asn Leu Ser Thr Val Leu Pro Thr Glu Gly Thr Leu
65                  70                  75                  80

Gln Glu Asn Val Ser Glu Ala Ser Lys Glu Val Ser Glu Lys Ala Lys
                85                  90                  95

Thr Leu Leu Ile Asp Arg Asp Leu Thr Glu Phe Ser Glu Leu Glu Tyr
            100                 105                 110

Ser Glu Met Gly Ser Ser Phe Ser Val Ser Pro Lys Ala Glu Ser Ala
        115                 120                 125

Val Ile Val Ala Asn Pro Arg Glu Glu Ile Ile Val Lys Asn Lys Asp
    130                 135                 140

Glu Glu Glu Lys Leu Val Ser Asn Asn Ile Leu His Asn Gln Gln Glu
145                 150                 155                 160

Leu Pro Thr Ala Leu Thr Lys Leu Val Lys Glu Asp Glu Val Val Ser
                165                 170                 175

Ser Glu Lys Ala Lys Asp Ser Phe Asn Glu Lys Arg Val Ala Val Glu
            180                 185                 190

Ala Pro Met Arg Glu Glu Tyr Ala Asp Phe Lys Pro Phe Glu Arg Val
        195                 200                 205

Trp Glu Val Lys Asp Ser Lys Glu Asp Ser Asp Met Leu Ala Ala Gly
    210                 215                 220

Gly Lys Ile Glu Ser Asn Leu Glu Ser Lys Val Asp Lys Lys Cys Phe
225                 230                 235                 240

Ala Asp Ser Leu Glu Gln Thr Asn His Glu Lys Asp Ser Glu Ser Ser
                245                 250                 255

Asn Asp Asp Thr Ser Phe Pro Ser Thr Pro Glu Gly Ile Lys Asp Arg
            260                 265                 270

Ser Gly Ala Tyr Ile Thr Cys Ala Pro Phe Asn Pro Ala Ala Thr Glu

        275                 280                 285

Ser Ile Ala Thr Asn Ile Phe Pro Leu Leu Gly Asp Pro Thr Ser Glu
    290                 295                 300

Asn Lys Thr Asp Glu Lys Lys Ile Glu Glu Lys Lys Ala Gln Ile Val
305                 310                 315                 320

Thr Glu Lys Asn Thr Ser Thr Lys Thr Ser Asn Pro Phe Leu Val Ala
                325                 330                 335

Ala Gln Asp Ser Glu Thr Asp Tyr Val Thr Thr Asp Asn Leu Thr Lys
            340                 345                 350

Val Thr Glu Glu Val Val Ala Asn Met Pro Glu Gly Leu Thr Pro Asp
        355                 360                 365

Leu Val Gln Glu Ala Cys Glu Ser Glu Leu Asn Glu Val Thr Gly Thr
    370                 375                 380

Lys Ile Ala Tyr Glu Thr Lys Met Asp Leu Val Gln Thr Ser Glu Val
385                 390                 395                 400

Met Gln Glu Ser Leu Tyr Pro Ala Ala Gln Leu Cys Pro Ser Phe Glu
                405                 410                 415

Glu Ser Glu Ala Thr Pro Ser Pro Val Leu Pro Asp Ile Val Met Glu
            420                 425                 430

Ala Pro Leu Asn Ser Ala Val Pro Ser Ala Gly Ala Ser Val Ile Gln
        435                 440                 445

Pro Ser Ser Ser Pro Leu Glu Ala Ser Ser Val Asn Tyr Glu Ser Ile
    450                 455                 460

Lys His Glu Pro Glu Asn Pro Pro Pro Tyr Glu Glu Ala Met Ser Val
465                 470                 475                 480

Ser Leu Lys Lys Val Ser Gly Ile Lys Glu Glu Ile Lys Glu Pro Glu
                485                 490                 495

Asn Ile Asn Ala Ala Leu Gln Glu Thr Glu Ala Pro Tyr Ile Ser Ile
            500                 505                 510

Ala Cys Asp Leu Ile Lys Glu Thr Lys Leu Ser Ala Glu Pro Ala Pro
        515                 520                 525

Asp Phe Ser Asp Tyr Ser Glu Met Ala Lys Val Glu Gln Pro Val Pro
    530                 535                 540

Asp His Ser Glu Leu Val Glu Asp Ser Ser Pro Asp Ser Glu Pro Val
545                 550                 555                 560

Asp Leu Phe Ser Asp Asp Ser Ile Pro Asp Val Pro Gln Lys Gln Asp
                565                 570                 575

Glu Thr Val Met Leu Val Lys Glu Ser Leu Thr Glu Thr Ser Phe Glu
            580                 585                 590

Ser Met Ile Glu Tyr Glu Asn Lys Glu Lys Leu Ser Ala Leu Pro Pro
        595                 600                 605

Glu Gly Gly Lys Pro Tyr Leu Glu Ser Phe Lys Leu Ser Leu Asp Asn
    610                 615                 620

Thr Lys Asp Thr Leu Leu Pro Asp Glu Val Ser Thr Leu Ser Lys Lys
625                 630                 635                 640

Glu Lys Ile Pro Leu Gln Met Glu Glu Leu Ser Thr Ala Val Tyr Ser
                645                 650                 655

Asn Asp Asp Leu Phe Ile Ser Lys Glu Ala Gln Ile Arg Glu Thr Glu
            660                 665                 670

Thr Phe Ser Asp Ser Ser Pro Ile Glu Ile Ile Asp Glu Phe Pro Thr
        675                 680                 685

Leu Ile Ser Ser Lys Thr Asp Ser Phe Ser Lys Leu Ala Arg Glu Tyr
    690                 695                 700

Thr Asp Leu Glu Val Ser His Lys Ser Glu Ile Ala Asn Ala Pro Asp
705                 710                 715                 720

Gly Ala Gly Ser Leu Pro Cys Thr Glu Leu Pro His Asp Leu Ser Leu
                725                 730                 735

Lys Asn Ile Gln Pro Lys Val Glu Glu Lys Ile Ser Phe Ser Asp Asp
            740                 745                 750

Phe Ser Lys Asn Gly Ser Ala Thr Ser Lys Val Leu Leu Leu Pro Pro
        755                 760                 765

Asp Val Ser Ala Leu Ala Thr Gln Ala Glu Ile Glu Ser Ile Val Lys
    770                 775                 780

Pro Lys Val Leu Val Lys Glu Ala Glu Lys Lys Leu Pro Ser Asp Thr
785                 790                 795                 800

Glu Lys Glu Asp Arg Ser Pro Ser Ala Ile Phe Ser Ala Glu Leu Ser

805                 810                 815

Lys Thr Ser

<210>  8

<211>  10

<212>  PRT

<213>  Mus musculus

<220>

<221>  BINDING

<222>  (1)..(10)

<223>  hypervariable part of heavy chain of 3A6

<400>  8

Gly Phe Asp Phe Arg Arg Asn Trp Met Ser
1               5                   10

<210>  9

<211>  17

<212>  PRT

<213>  Mus musculus

<220>

<221>  BINDING

<222>  (1)..(17)

<223>  hypervariable part of heavy chain of 3A6

<400>  9

Glu Ile Asn Pro Asp Ser Ser Lys Ile Asn Tyr Thr Pro Ser Leu Lys
1               5                   10                  15

Asp

<210>  10

<211>  9

<212>  PRT

<213>  Mus musculus

<220>

<221>  BINDING

<222>  (1)..(9)

<223>  hypervariable part of heavy chain of 3A6

<400>  10

Pro Val Trp Met Tyr Ala Met Asp Tyr
1               5

<210>  11

<211>  16

<212>  PRT

<213>  Mus musculus

<220>

<221>  BINDING

<222>  (1)..(16)

<223>  hypervariable part of light chain of 3A6

<400>  11

Lys Ser Ser Gln Ser Leu Leu His Ser Asp Gly Lys Thr Tyr Leu Asn
1               5                   10                  15

<210>  12

<211>  7

<212>  PRT

<213>  Mus musculus

<220>

<221>  BINDING

<222>  (1)..(7)

<223>  hypervariable part of light chain of 3A6

<400>  12

Leu Val Ser Lys Leu Asp Ser
1               5

<210>  13

<211>  9

<212>  PRT

<213>  Mus musculus

<220>

<221>  BINDING

<222>  (1)..(9)

<223>  hypervariable part of light chain of 3A6

<400>  13

Trp Gln Gly Thr His Phe Pro Gln Thr
1               5

<210>  14

<211>  30

<212>  DNA

<213>  Mus musculus

<220>

<221>  misc_binding

<222>  (1)..(30)

<223>  DNA-CDR-H1-3A6

<400>  14
ggattcgatt ttagaagaaa ttggatgagt

<210>  15

<211>  51

<212>  DNA

<213>  Mus musculus

<220>

<221>  misc_binding

<222>  (1)..(51)

<223>  DNA-CDR-H2-3A6

<400>  15
gaaattaatc cagatagcag taagataaac tatacgccat ctctaaagga t                51

<210>  16

<211>  27

<212>  DNA

<213>  Mus musculus

<220>

<221>  misc_binding

<222>  (1)..(27)

<223>  DNA-CDR-H3-3A6

<400>  16
ccggtctgga tgtatgctat ggactac                                          27

<210>  17

<211>  48

<212>  DNA

<213>  Mus musculus

<220>

<221>  misc_binding

<222>  (1)..(48)

<223>  DNA-CDR'1-3A6

<400>  17

aagtcaagtc agagcctctt gcatagtgat ggaaagacat atttgaat 48

<210> 18

<211> 21

<212> DNA

<213> Mus musculus

<220>

<221> misc_binding

<222> (1)..(21)

<223> DNA-CDR'2-3A6

<400> 18
ctggtgtcta aactggactc t 21

<210> 19

<211> 27

<212> DNA

<213>  Mus musculus

<220>

<221>  misc_binding

<222>  (1)..(27)

<223>  DNA-CDR'3-3A6

<400>  19
tggcaaggta cacattttcc tcagacg                                          27

<210>  20

<211>  54

<212>  DNA

<213>  Mus musculus

<220>

<221>  CDS

<222>  (1)..(54)

<223>  leader sequence for heavy chain of 3A6

<400>  20
atg gat ttt ggg ctg att ttt ttt att gtt ggt ctt tta aaa ggg gtc        48
Met Asp Phe Gly Leu Ile Phe Phe Ile Val Gly Leu Leu Lys Gly Val
1               5                   10                  15

cag tgt                                                                54
Gln Cys

<210>  21

<211>  18

<212>  PRT

<213>  Mus musculus

<400>  21

Met Asp Phe Gly Leu Ile Phe Phe Ile Val Gly Leu Leu Lys Gly Val
1               5                   10                  15

Gln Cys

<210> 22

<211> 57

<212> DNA

<213> Mus musculus

<220>

<221> CDS

<222> (1)..(57)

<223> leader sequence for 3A6-light chain

<400> 22
atg agt cct gcc cag ttc ctg ttt ctg tta gtg ctc tgg att cgg gaa 48
Met Ser Pro Ala Gln Phe Leu Phe Leu Leu Val Leu Trp Ile Arg Glu
1               5                   10                  15

acc agc ggt 57
Thr Ser Gly

<210>  23

<211>  19

<212>  PRT

<213>  Mus musculus

<400>  23

Met Ser Pro Ala Gln Phe Leu Phe Leu Leu Val Leu Trp Ile Arg Glu
1               5                   10                  15

Thr Ser Gly

<210>  24

<211>  181

<212>  PRT

<213>  Homo sapiens

<220>

<221>  PEPTIDE

<222>  (1)..(181)

<223>  human Nig-D20

<400>  24

Gly Thr Lys Ile Ala Tyr Glu Thr Lys Met Asp Leu Val Gln Thr Ser
1               5                   10                  15

Glu Val Met Gln Glu Ser Leu Tyr Pro Ala Ala Gln Leu Cys Pro Ser
            20                  25                  30

Phe Glu Glu Ser Glu Ala Thr Pro Ser Pro Val Leu Pro Asp Ile Val
        35                  40                  45

Met Glu Ala Pro Leu Asn Ser Ala Val Pro Ser Ala Gly Ala Ser Val
    50                  55                  60

Ile Gln Pro Ser Ser Ser Pro Leu Glu Ala Ser Ser Val Asn Tyr Glu
65                  70                  75                  80

Ser Ile Lys His Glu Pro Glu Asn Pro Pro Pro Tyr Glu Glu Ala Met
                85                  90                  95

Ser Val Ser Leu Lys Lys Val Ser Gly Ile Lys Glu Glu Ile Lys Glu
            100                 105                 110

Pro Glu Asn Ile Asn Ala Ala Leu Gln Glu Thr Glu Ala Pro Tyr Ile
        115                 120                 125

Ser Ile Ala Cys Asp Leu Ile Lys Glu Thr Lys Leu Ser Ala Glu Pro
    130                 135                 140

Ala Pro Asp Phe Ser Asp Tyr Ser Glu Met Ala Lys Val Glu Gln Pro
145                 150                 155                 160

Val Pro Asp His Ser Glu Leu Val Glu Asp Ser Ser Pro Asp Ser Glu
                165                 170                 175

Pro Val Asp Leu Phe
            180

<210>  25

<211>  3492

<212>  DNA

<213>  Rattus norvegicus

<220>

<221>  CDS

<222>  (1)..(3492)

<223>  rat NogoA

<400>  25
atg gaa gac ata gac cag tcg tcg ctg gtc tcc tcg tcc acg gac agc      48
Met Glu Asp Ile Asp Gln Ser Ser Leu Val Ser Ser Ser Thr Asp Ser
1               5                   10                  15

ccg ccc cgg cct ccg ccc gcc ttc aag tac cag ttc gtg acg gag ccc      96
Pro Pro Arg Pro Pro Pro Ala Phe Lys Tyr Gln Phe Val Thr Glu Pro
            20                  25                  30

gag gac gag gag gac gag gag gag gag gag gac gag gag gag gac gac      144
Glu Asp Glu Glu Asp Glu Glu Glu Glu Glu Asp Glu Glu Glu Asp Asp
        35                  40                  45

gag gac cta gag gaa ctg gag gtg ctg gag agg aag ccc gca gcc ggg      192
Glu Asp Leu Glu Glu Leu Glu Val Leu Glu Arg Lys Pro Ala Ala Gly
    50                  55                  60

ctg tcc gca gct gcg gtg ccg ccc gcc gcc gcc gcg ccg ctg ctg gac      240
Leu Ser Ala Ala Ala Val Pro Pro Ala Ala Ala Ala Pro Leu Leu Asp
65                  70                  75                  80

ttc agc agc gac tcg gtg ccc ccc gcg ccc cgc ggg ccg ctg ccg gcc 288
Phe Ser Ser Asp Ser Val Pro Pro Ala Pro Arg Gly Pro Leu Pro Ala
                85                  90                  95

gcg ccc cct gcc gct cct gag agg cag cca tcc tgg gaa cgc agc ccc 336
Ala Pro Pro Ala Ala Pro Glu Arg Gln Pro Ser Trp Glu Arg Ser Pro
            100                 105                 110

gcg gcg ccc gcg cca tcc ctg ccg ccc gct gcc gca gtc ctg ccc tcc 384
Ala Ala Pro Ala Pro Ser Leu Pro Pro Ala Ala Ala Val Leu Pro Ser
        115                 120                 125

aag ctc cca gag gac gac gag cct ccg gcg agg ccc ccg cct ccg ccg 432
Lys Leu Pro Glu Asp Asp Glu Pro Pro Ala Arg Pro Pro Pro Pro Pro
    130                 135                 140

cca gcc ggc gcg agc ccc ctg gcg gag ccc gcc gcg ccc cct tcc acg 480
Pro Ala Gly Ala Ser Pro Leu Ala Glu Pro Ala Ala Pro Pro Ser Thr
145                 150                 155                 160

ccg gcc gcg ccc aag cgc agg ggc tcc ggc tca gtg gat gag acc ctt 528
Pro Ala Ala Pro Lys Arg Arg Gly Ser Gly Ser Val Asp Glu Thr Leu
                165                 170                 175

ttt gct ctt cct gct gca tct gag cct gtg ata ccc tcc tct gca gaa 576
Phe Ala Leu Pro Ala Ala Ser Glu Pro Val Ile Pro Ser Ser Ala Glu
            180                 185                 190

aaa att atg gat ttg atg gag cag cca ggt aac act gtt tcg tct ggt 624
Lys Ile Met Asp Leu Met Glu Gln Pro Gly Asn Thr Val Ser Ser Gly
        195                 200                 205

caa gag gat ttc cca tct gtc ctg ctt gaa act gct gcc tct ctt cct 672

Gln Glu Asp Phe Pro Ser Val Leu Leu Glu Thr Ala Ala Ser Leu Pro
    210                 215                 220

tct cta tct cct ctc tca act gtt tct ttt aaa gaa cat gga tac ctt      720
Ser Leu Ser Pro Leu Ser Thr Val Ser Phe Lys Glu His Gly Tyr Leu
225                 230                 235                 240

ggt aac tta tca gca gtg tca tcc tca gaa gga aca att gaa gaa act      768
Gly Asn Leu Ser Ala Val Ser Ser Ser Glu Gly Thr Ile Glu Glu Thr
                245                 250                 255

tta aat gaa gct tct aaa gag ttg cca gag agg gca aca aat cca ttt      816
Leu Asn Glu Ala Ser Lys Glu Leu Pro Glu Arg Ala Thr Asn Pro Phe
            260                 265                 270

gta aat aga gat tta gca gaa ttt tca gaa tta gaa tat tca gaa atg      864
Val Asn Arg Asp Leu Ala Glu Phe Ser Glu Leu Glu Tyr Ser Glu Met
        275                 280                 285

gga tca tct ttt aaa ggc tcc cca aaa gga gag tca gcc ata tta gta      912
Gly Ser Ser Phe Lys Gly Ser Pro Lys Gly Glu Ser Ala Ile Leu Val
    290                 295                 300

gaa aac act aag gaa gaa gta att gtg agg agt aaa gac aaa gag gat      960
Glu Asn Thr Lys Glu Glu Val Ile Val Arg Ser Lys Asp Lys Glu Asp
305                 310                 315                 320

tta gtt tgt agt gca gcc ctt cac agt cca caa gaa tca cct gtg ggt     1008
Leu Val Cys Ser Ala Ala Leu His Ser Pro Gln Glu Ser Pro Val Gly
                325                 330                 335

aaa gaa gac aga gtt gtg tct cca gaa aag aca atg gac att ttt aat     1056
Lys Glu Asp Arg Val Val Ser Pro Glu Lys Thr Met Asp Ile Phe Asn

            340                 345                 350

gaa atg cag atg tca gta gta gca cct gtg agg gaa gag tat gca gac 1104
Glu Met Gln Met Ser Val Val Ala Pro Val Arg Glu Glu Tyr Ala Asp
        355                 360                 365

ttt aag cca ttt gaa caa gca tgg gaa gtg aaa gat act tat gag gga 1152
Phe Lys Pro Phe Glu Gln Ala Trp Glu Val Lys Asp Thr Tyr Glu Gly
    370                 375                 380

agt agg gat gtg ctg gct gct aga gct aat gtg gaa agt aaa gtg gac 1200
Ser Arg Asp Val Leu Ala Ala Arg Ala Asn Val Glu Ser Lys Val Asp
385                 390                 395                 400

aga aaa tgc ttg gaa gat agc ctg gag caa aaa agt ctt ggg aag gat 1248
Arg Lys Cys Leu Glu Asp Ser Leu Glu Gln Lys Ser Leu Gly Lys Asp
                405                 410                 415

agt gaa ggc aga aat gag gat gct tct ttc ccc agt acc cca gaa cct 1296
Ser Glu Gly Arg Asn Glu Asp Ala Ser Phe Pro Ser Thr Pro Glu Pro
            420                 425                 430

gtg aag gac agc tcc aga gca tat att acc tgt gct tcc ttt acc tca 1344
Val Lys Asp Ser Ser Arg Ala Tyr Ile Thr Cys Ala Ser Phe Thr Ser
        435                 440                 445

gca acc gaa agc acc aca gca aac act ttc cct ttg tta gaa gat cat 1392
Ala Thr Glu Ser Thr Thr Ala Asn Thr Phe Pro Leu Leu Glu Asp His
    450                 455                 460

act tca gaa aat aaa aca gat gaa aaa aaa ata gaa gaa agg aag gcc 1440
Thr Ser Glu Asn Lys Thr Asp Glu Lys Lys Ile Glu Glu Arg Lys Ala
465                 470                 475                 480

caa att ata aca gag aag act agc ccc aaa acg tca aat cct ttc ctt     1488
Gln Ile Ile Thr Glu Lys Thr Ser Pro Lys Thr Ser Asn Pro Phe Leu
                485                 490                 495

gta gca gta cag gat tct gag gca gat tat gtt aca aca gat acc tta     1536
Val Ala Val Gln Asp Ser Glu Ala Asp Tyr Val Thr Thr Asp Thr Leu
            500                 505                 510

tca aag gtg act gag gca gca gtg tca aac atg cct gaa ggt ctg acg     1584
Ser Lys Val Thr Glu Ala Ala Val Ser Asn Met Pro Glu Gly Leu Thr
        515                 520                 525

cca gat tta gtt cag gaa gca tgt gaa agt gaa ctg aat gaa gcc aca     1632
Pro Asp Leu Val Gln Glu Ala Cys Glu Ser Glu Leu Asn Glu Ala Thr
    530                 535                 540

ggt aca aag att gct tat gaa aca aaa gtg gac ttg gtc caa aca tca     1680
Gly Thr Lys Ile Ala Tyr Glu Thr Lys Val Asp Leu Val Gln Thr Ser
545                 550                 555                 560

gaa gct ata caa gaa tca ctt tac ccc aca gca cag ctt tgc cca tca     1728
Glu Ala Ile Gln Glu Ser Leu Tyr Pro Thr Ala Gln Leu Cys Pro Ser
                565                 570                 575

ttt gag gaa gct gaa gca act ccg tca cca gtt ttg cct gat att gtt     1776
Phe Glu Glu Ala Glu Ala Thr Pro Ser Pro Val Leu Pro Asp Ile Val
            580                 585                 590

atg gaa gca cca tta aat tct ctc ctt cca agc gct ggt gct tct gta     1824
Met Glu Ala Pro Leu Asn Ser Leu Leu Pro Ser Ala Gly Ala Ser Val
        595                 600                 605

gtg cag ccc agt gta tcc cca ctg gaa gca cct cct cca gtt agt tat     1872
Val Gln Pro Ser Val Ser Pro Leu Glu Ala Pro Pro Pro Val Ser Tyr
    610                 615                 620

gac agt ata aag ctt gag cct gaa aac ccc cca cca tat gaa gaa gcc     1920
Asp Ser Ile Lys Leu Glu Pro Glu Asn Pro Pro Pro Tyr Glu Glu Ala
625                 630                 635                 640

atg aat gta gca cta aaa gct ttg gga aca aag gaa gga ata aaa gag     1968
Met Asn Val Ala Leu Lys Ala Leu Gly Thr Lys Glu Gly Ile Lys Glu
                645                 650                 655

cct gaa agt ttt aat gca gct gtt cag gaa aca gaa gct cct tat ata     2016
Pro Glu Ser Phe Asn Ala Ala Val Gln Glu Thr Glu Ala Pro Tyr Ile
            660                 665                 670

tcc att gcg tgt gat tta att aaa gaa aca aag ctc tcc act gag cca     2064
Ser Ile Ala Cys Asp Leu Ile Lys Glu Thr Lys Leu Ser Thr Glu Pro
        675                 680                 685

agt cca gat ttc tct aat tat tca gaa ata gca aaa ttc gag aag tcg     2112
Ser Pro Asp Phe Ser Asn Tyr Ser Glu Ile Ala Lys Phe Glu Lys Ser
    690                 695                 700

gtg ccc gaa cac gct gag cta gtg gag gat tcc tca cct gaa tct gaa     2160
Val Pro Glu His Ala Glu Leu Val Glu Asp Ser Ser Pro Glu Ser Glu
705                 710                 715                 720

cca gtt gac tta ttt agt gat gat tcg att cct gaa gtc cca caa aca     2208
Pro Val Asp Leu Phe Ser Asp Asp Ser Ile Pro Glu Val Pro Gln Thr
                725                 730                 735

caa gag gag gct gtg atg ctc atg aag gag agt ctc act gaa gtg tct     2256

Gln Glu Glu Ala Val Met Leu Met Lys Glu Ser Leu Thr Glu Val Ser
            740                 745                 750

gag aca gta gcc cag cac aaa gag gag aga ctt agt gcc tca cct cag      2304
Glu Thr Val Ala Gln His Lys Glu Glu Arg Leu Ser Ala Ser Pro Gln
        755                 760                 765

gag cta gga aag cca tat tta gag tct ttt cag ccc aat tta cat agt      2352
Glu Leu Gly Lys Pro Tyr Leu Glu Ser Phe Gln Pro Asn Leu His Ser
    770                 775                 780

aca aaa gat gct gca tct aat gac att cca aca ttg acc aaa aag gag      2400
Thr Lys Asp Ala Ala Ser Asn Asp Ile Pro Thr Leu Thr Lys Lys Glu
785                 790                 795                 800

aaa att tct ttg caa atg gaa gag ttt aat act gca att tat tca aat      2448
Lys Ile Ser Leu Gln Met Glu Glu Phe Asn Thr Ala Ile Tyr Ser Asn
                805                 810                 815

gat gac tta ctt tct tct aag gaa gac aaa ata aaa gaa agt gaa aca      2496
Asp Asp Leu Leu Ser Ser Lys Glu Asp Lys Ile Lys Glu Ser Glu Thr
            820                 825                 830

ttt tca gat tca tct ccg att gag ata ata gat gaa ttt ccc acg ttt      2544
Phe Ser Asp Ser Ser Pro Ile Glu Ile Ile Asp Glu Phe Pro Thr Phe
        835                 840                 845

gtc agt gct aaa gat gat tct cct aaa tta gcc aag gag tac act gat      2592
Val Ser Ala Lys Asp Asp Ser Pro Lys Leu Ala Lys Glu Tyr Thr Asp
    850                 855                 860

cta gaa gta tcc gac aaa agt gaa att gct aat atc caa agc ggg gca      2640
Leu Glu Val Ser Asp Lys Ser Glu Ile Ala Asn Ile Gln Ser Gly Ala

865                 870                 875                 880

gat tca ttg cct tgc tta gaa ttg ccc tgt gac ctt tct ttc aag aat     2688
Asp Ser Leu Pro Cys Leu Glu Leu Pro Cys Asp Leu Ser Phe Lys Asn
                885                 890                 895

ata tat cct aaa gat gaa gta cat gtt tca gat gaa ttc tcc gaa aat     2736
Ile Tyr Pro Lys Asp Glu Val His Val Ser Asp Glu Phe Ser Glu Asn
            900                 905                 910

agg tcc agt gta tct aag gca tcc ata tcg cct tca aat gtc tct gct     2784
Arg Ser Ser Val Ser Lys Ala Ser Ile Ser Pro Ser Asn Val Ser Ala
        915                 920                 925

ttg gaa cct cag aca gaa atg ggc agc ata gtt aaa tcc aaa tca ctt     2832
Leu Glu Pro Gln Thr Glu Met Gly Ser Ile Val Lys Ser Lys Ser Leu
    930                 935                 940

acg aaa gaa gca gag aaa aaa ctt cct tct gac aca gag aaa gag gac     2880
Thr Lys Glu Ala Glu Lys Lys Leu Pro Ser Asp Thr Glu Lys Glu Asp
945                 950                 955                 960

aga tcc ctg tca gct gta ttg tca gca gag ctg agt aaa act tca gtt     2928
Arg Ser Leu Ser Ala Val Leu Ser Ala Glu Leu Ser Lys Thr Ser Val
                965                 970                 975

gtt gac ctc ctc tac tgg aga gac att aag aag act gga gtg gtg ttt     2976
Val Asp Leu Leu Tyr Trp Arg Asp Ile Lys Lys Thr Gly Val Val Phe
            980                 985                 990

ggt gcc agc tta ttc ctg ctg ctg  tct ctg aca gtg ttc  agc att gtc     3024
Gly Ala Ser Leu Phe Leu Leu Leu  Ser Leu Thr Val Phe  Ser Ile Val
        995                 1000                1005

agt gta acg gcc tac att gcc ttg gcc ctg ctc tcg gtg act atc 3069
Ser Val Thr Ala Tyr Ile Ala Leu Ala Leu Leu Ser Val Thr Ile
    1010                1015                1020

agc ttt agg ata tat aag ggc gtg atc cag gct atc cag aaa tca 3114
Ser Phe Arg Ile Tyr Lys Gly Val Ile Gln Ala Ile Gln Lys Ser
    1025                1030                1035

gat gaa ggc cac cca ttc agg gca tat tta gaa tct gaa gtt gct 3159
Asp Glu Gly His Pro Phe Arg Ala Tyr Leu Glu Ser Glu Val Ala
    1040                1045                1050

ata tca gag gaa ttg gtt cag aaa tac agt aat tct gct ctt ggt 3204
Ile Ser Glu Glu Leu Val Gln Lys Tyr Ser Asn Ser Ala Leu Gly
    1055                1060                1065

cat gtg aac agc aca ata aaa gaa ctg agg cgg ctt ttc tta gtt 3249
His Val Asn Ser Thr Ile Lys Glu Leu Arg Arg Leu Phe Leu Val
    1070                1075                1080

gat gat tta gtt gat tcc ctg aag ttt gca gtg ttg atg tgg gtg 3294
Asp Asp Leu Val Asp Ser Leu Lys Phe Ala Val Leu Met Trp Val
    1085                1090                1095

ttt act tat gtt ggt gcc ttg ttc aat ggt ctg aca cta ctg att 3339
Phe Thr Tyr Val Gly Ala Leu Phe Asn Gly Leu Thr Leu Leu Ile
    1100                1105                1110

tta gct ctg atc tca ctc ttc agt att cct gtt att tat gaa cgg 3384
Leu Ala Leu Ile Ser Leu Phe Ser Ile Pro Val Ile Tyr Glu Arg
    1115                1120                1125

cat cag gtg cag ata gat cat tat cta gga ctt gca aac aag agt 3429
His Gln Val Gln Ile Asp His Tyr Leu Gly Leu Ala Asn Lys Ser
    1130                1135                1140

gtt aag gat gcc atg gcc aaa atc caa gca aaa atc cct gga ttg 3474
Val Lys Asp Ala Met Ala Lys Ile Gln Ala Lys Ile Pro Gly Leu
    1145                1150                1155

aag cgc aaa gca gat tga 3492
Lys Arg Lys Ala Asp
    1160

<210> 26

<211> 1163

<212> PRT

<213> Rattus norvegicus

<400> 26

Met Glu Asp Ile Asp Gln Ser Ser Leu Val Ser Ser Ser Thr Asp Ser
1               5                   10                  15

Pro Pro Arg Pro Pro Pro Ala Phe Lys Tyr Gln Phe Val Thr Glu Pro
            20                  25                  30

Glu Asp Glu Glu Asp Glu Glu Glu Glu Glu Asp Glu Glu Glu Asp Asp
        35                  40                  45

Glu Asp Leu Glu Glu Leu Glu Val Leu Glu Arg Lys Pro Ala Ala Gly
    50                  55                  60

Leu Ser Ala Ala Ala Val Pro Pro Ala Ala Ala Ala Pro Leu Leu Asp
65                  70                  75                  80

Phe Ser Ser Asp Ser Val Pro Pro Ala Pro Arg Gly Pro Leu Pro Ala
                85                  90                  95

Ala Pro Pro Ala Ala Pro Glu Arg Gln Pro Ser Trp Glu Arg Ser Pro
            100                 105                 110

Ala Ala Pro Ala Pro Ser Leu Pro Pro Ala Ala Ala Val Leu Pro Ser
        115                 120                 125

Lys Leu Pro Glu Asp Asp Glu Pro Pro Ala Arg Pro Pro Pro Pro Pro
    130                 135                 140

Pro Ala Gly Ala Ser Pro Leu Ala Glu Pro Ala Ala Pro Pro Ser Thr
145                 150                 155                 160

Pro Ala Ala Pro Lys Arg Arg Gly Ser Gly Ser Val Asp Glu Thr Leu

165                 170                 175

Phe Ala Leu Pro Ala Ala Ser Glu Pro Val Ile Pro Ser Ser Ala Glu
            180                 185                 190

Lys Ile Met Asp Leu Met Glu Gln Pro Gly Asn Thr Val Ser Ser Gly
        195                 200                 205

Gln Glu Asp Phe Pro Ser Val Leu Leu Glu Thr Ala Ala Ser Leu Pro
    210                 215                 220

Ser Leu Ser Pro Leu Ser Thr Val Ser Phe Lys Glu His Gly Tyr Leu
225                 230                 235                 240

Gly Asn Leu Ser Ala Val Ser Ser Ser Glu Gly Thr Ile Glu Glu Thr
                245                 250                 255

Leu Asn Glu Ala Ser Lys Glu Leu Pro Glu Arg Ala Thr Asn Pro Phe
            260                 265                 270

Val Asn Arg Asp Leu Ala Glu Phe Ser Glu Leu Glu Tyr Ser Glu Met
        275                 280                 285

Gly Ser Ser Phe Lys Gly Ser Pro Lys Gly Glu Ser Ala Ile Leu Val
    290                 295                 300

Glu Asn Thr Lys Glu Glu Val Ile Val Arg Ser Lys Asp Lys Glu Asp
305                 310                 315                 320

Leu Val Cys Ser Ala Ala Leu His Ser Pro Gln Glu Ser Pro Val Gly
                325                 330                 335

Lys Glu Asp Arg Val Val Ser Pro Glu Lys Thr Met Asp Ile Phe Asn
            340                 345                 350

Glu Met Gln Met Ser Val Val Ala Pro Val Arg Glu Glu Tyr Ala Asp
        355                 360                 365

Phe Lys Pro Phe Glu Gln Ala Trp Glu Val Lys Asp Thr Tyr Glu Gly
    370                 375                 380

Ser Arg Asp Val Leu Ala Ala Arg Ala Asn Val Glu Ser Lys Val Asp
385                 390                 395                 400

Arg Lys Cys Leu Glu Asp Ser Leu Glu Gln Lys Ser Leu Gly Lys Asp
                405                 410                 415

Ser Glu Gly Arg Asn Glu Asp Ala Ser Phe Pro Ser Thr Pro Glu Pro
            420                 425                 430

Val Lys Asp Ser Ser Arg Ala Tyr Ile Thr Cys Ala Ser Phe Thr Ser
        435                 440                 445

Ala Thr Glu Ser Thr Thr Ala Asn Thr Phe Pro Leu Leu Glu Asp His
    450                 455                 460

Thr Ser Glu Asn Lys Thr Asp Glu Lys Lys Ile Glu Glu Arg Lys Ala
465                 470                 475                 480

Gln Ile Ile Thr Glu Lys Thr Ser Pro Lys Thr Ser Asn Pro Phe Leu
                485                 490                 495

Val Ala Val Gln Asp Ser Glu Ala Asp Tyr Val Thr Thr Asp Thr Leu
            500                 505                 510

Ser Lys Val Thr Glu Ala Ala Val Ser Asn Met Pro Glu Gly Leu Thr
        515                 520                 525

Pro Asp Leu Val Gln Glu Ala Cys Glu Ser Glu Leu Asn Glu Ala Thr
    530                 535                 540

Gly Thr Lys Ile Ala Tyr Glu Thr Lys Val Asp Leu Val Gln Thr Ser
545                 550                 555                 560

Glu Ala Ile Gln Glu Ser Leu Tyr Pro Thr Ala Gln Leu Cys Pro Ser
                565                 570                 575

Phe Glu Glu Ala Glu Ala Thr Pro Ser Pro Val Leu Pro Asp Ile Val
            580                 585                 590

Met Glu Ala Pro Leu Asn Ser Leu Leu Pro Ser Ala Gly Ala Ser Val
        595                 600                 605

Val Gln Pro Ser Val Ser Pro Leu Glu Ala Pro Pro Pro Val Ser Tyr
    610                 615                 620

Asp Ser Ile Lys Leu Glu Pro Glu Asn Pro Pro Pro Tyr Glu Glu Ala
625                 630                 635                 640

Met Asn Val Ala Leu Lys Ala Leu Gly Thr Lys Glu Gly Ile Lys Glu
                645                 650                 655

Pro Glu Ser Phe Asn Ala Ala Val Gln Glu Thr Glu Ala Pro Tyr Ile
            660                 665                 670

Ser Ile Ala Cys Asp Leu Ile Lys Glu Thr Lys Leu Ser Thr Glu Pro
        675                 680                 685

Ser Pro Asp Phe Ser Asn Tyr Ser Glu Ile Ala Lys Phe Glu Lys Ser

    690                 695                 700

Val Pro Glu His Ala Glu Leu Val Glu Asp Ser Ser Pro Glu Ser Glu
705                 710                 715                 720

Pro Val Asp Leu Phe Ser Asp Asp Ser Ile Pro Glu Val Pro Gln Thr
                725                 730                 735

Gln Glu Glu Ala Val Met Leu Met Lys Glu Ser Leu Thr Glu Val Ser
            740                 745                 750

Glu Thr Val Ala Gln His Lys Glu Glu Arg Leu Ser Ala Ser Pro Gln
        755                 760                 765

Glu Leu Gly Lys Pro Tyr Leu Glu Ser Phe Gln Pro Asn Leu His Ser
    770                 775                 780

Thr Lys Asp Ala Ala Ser Asn Asp Ile Pro Thr Leu Thr Lys Lys Glu
785                 790                 795                 800

Lys Ile Ser Leu Gln Met Glu Glu Phe Asn Thr Ala Ile Tyr Ser Asn
                805                 810                 815

Asp Asp Leu Leu Ser Ser Lys Glu Asp Lys Ile Lys Glu Ser Glu Thr
            820                 825                 830

Phe Ser Asp Ser Ser Pro Ile Glu Ile Ile Asp Glu Phe Pro Thr Phe
        835                 840                 845

Val Ser Ala Lys Asp Asp Ser Pro Lys Leu Ala Lys Glu Tyr Thr Asp
    850                 855                 860

Leu Glu Val Ser Asp Lys Ser Glu Ile Ala Asn Ile Gln Ser Gly Ala
865                 870                 875                 880

Asp Ser Leu Pro Cys Leu Glu Leu Pro Cys Asp Leu Ser Phe Lys Asn
                885                 890                 895

Ile Tyr Pro Lys Asp Glu Val His Val Ser Asp Glu Phe Ser Glu Asn
            900                 905                 910

Arg Ser Ser Val Ser Lys Ala Ser Ile Ser Pro Ser Asn Val Ser Ala
        915                 920                 925

Leu Glu Pro Gln Thr Glu Met Gly Ser Ile Val Lys Ser Lys Ser Leu
    930                 935                 940

Thr Lys Glu Ala Glu Lys Lys Leu Pro Ser Asp Thr Glu Lys Glu Asp
945                 950                 955                 960

Arg Ser Leu Ser Ala Val Leu Ser Ala Glu Leu Ser Lys Thr Ser Val
                965                 970                 975

Val Asp Leu Leu Tyr Trp Arg Asp Ile Lys Lys Thr Gly Val Val Phe
            980                 985                 990

Gly Ala Ser Leu Phe Leu Leu Leu  Ser Leu Thr Val Phe  Ser Ile Val
        995                 1000                 1005

Ser Val  Thr Ala Tyr Ile Ala  Leu Ala Leu Leu Ser  Val Thr Ile
    1010                 1015                 1020

Ser Phe  Arg Ile Tyr Lys Gly  Val Ile Gln Ala Ile  Gln Lys Ser
    1025                 1030                 1035

Asp Glu  Gly His Pro Phe Arg  Ala Tyr Leu Glu Ser  Glu Val Ala
    1040                 1045                 1050

Ile Ser  Glu Glu Leu Val Gln  Lys Tyr Ser Asn Ser  Ala Leu Gly
    1055                 1060                 1065

His Val  Asn Ser Thr Ile Lys  Glu Leu Arg Arg Leu  Phe Leu Val
    1070                 1075                 1080

Asp Asp Leu Val Asp Ser Leu Lys Phe Ala Val Leu Met Trp Val
    1085                1090                1095

Phe Thr Tyr Val Gly Ala Leu Phe Asn Gly Leu Thr Leu Leu Ile
    1100                1105                1110

Leu Ala Leu Ile Ser Leu Phe Ser Ile Pro Val Ile Tyr Glu Arg
    1115                1120                1125

His Gln Val Gln Ile Asp His Tyr Leu Gly Leu Ala Asn Lys Ser
    1130                1135                1140

Val Lys Asp Ala Met Ala Lys Ile Gln Ala Lys Ile Pro Gly Leu
    1145                1150                1155

Lys Arg Lys Ala Asp
    1160

<210> 27

<211> 25

<212> PRT

<213> Rattus norvegicus

<220>

<221>  PEPTIDE

<222>  (1)..(25)

<223>  rat PEP4

<400>  27

Glu Glu Leu Val Gln Lys Tyr Ser Asn Ser Ala Leu Gly His Val Asn
1               5                   10                  15

Ser Thr Ile Lys Glu Leu Arg Arg Leu
            20                  25

<210>  28

<211>  17

<212>  PRT

<213>  Artificial Sequence

<220>

<223>  PRO/SER rich peptide

<220>

<221>  PEPTIDE

<222>  (1)..(17)

<223>  Synthetic peptide

<400>  28

Pro Ser Ser Pro Pro Pro Ser Ser Pro Pro Pro Ser Ser Pro Pro Pro
1               5                   10                  15

Ser

<210>  29

<211>  25

<212>  DNA

<213>  Artificial Sequence

<220>

<223>  CA-NA-2F

<220>

<221>  primer_bind

<222>  (1)..(25)

<223>  CA-NA-2F primer

<400>  29
aagcaccatt gaattctgca gttcc                                          25

<210>  30

<211>  28

<212>  DNA

<213>  Artificial Sequence

<220>

<223>  CA-NA-3R

<220>

<221>  primer_bind

<222>  (1)..(28)

<223>

<400>  30
aactgcagta ctgagctcct ccatctgc                                          28

<210>  31

<211>  33

<212>  DNA

<213>  Artificial Sequence

<220>

<223>  forward 5'

<220>

<221>  primer_bind

<222>  (1)..(33)

<223>  forward primer

<400>  31
gtcgcggatc catggagacc ctttttgctc ttc                                    33

<210>  32

<211>  27

<212>  DNA

<213>  Artificial Sequence

<220>

<223>  reverse 5'

<220>

<221>  primer_bind

<222>  (1)..(27)

<223>  reverse primer

<400>  32
gttctcgagt tatgaagttt tactcag                                          27

<210>  33

<211>  29

<212>  DNA

<213>  Artificial Sequence

<220>

<223>  forward 5'-1

<220>

<221>  primer_bind

<222>  (1)..(29)

<223>  primer

<400>  33
gtgcggatcc atggatttga aggagcagc                                        29

<210>  34

<211>  28

<212>  DNA

<213>  Artificial Sequence

<220>

<223>  reverse 5'-1

<220>

<221>  primer_bind

<222>  (1)..(28)

<223>  primer

<400>  34
gtttctcgag tgaagtttta ttcagctc                                          28

<210>  35

<211>  20

<212>  DNA

<213>  Artificial Sequence

<220>

<223>  5' primer

<220>

<221>  primer_bind

<222>  (1)..(20)

<223>  primer

<400>  35
tccaccccgg ccgcgcccaa                                                    20

<210>  36

<211>  22

<212>  DNA

<213>  Artificial Sequence

<220>

<223>  5' primer 2

<220>

<221>  primer_bind

<222>  (1)..(22)

<223>  primer

<400>  36
aatgatgggc aaagctgtgc tg                                                22

<210>  37

<211>  24

<212>  DNA

<213>  Artificial Sequence

<220>

<223>  3' primer

<220>

<221>  primer_bind

<222>  (1)..(24)

<223>  primer

<400>  37
ggtacaaaga ttgcttatga aaca                                              24

<210>  38

<211>  22

<212>  DNA

<213>  Artificial Sequence

<220>

<223>  3' primer 2

<220>

<221>  primer_bind

<222>  (1)..(22)

<223>  primer

<400>  38
agcagggcca aggcaatgta gg                                                22

<210>  39

<211>  28

<212>  DNA

<213>  Artificial Sequence

<220>

<223>  5'-VL leader

<220>

<221>  primer_bind

<222>  (1)..(28)

<223>  primer

<400>  39
aatatgagtc ctgcccagtt cctgtttc                                          28

<210>  40

<211>  32

<212>  DNA

<213>  Artificial Sequence

<220>

<223>  3'-Ck

<220>

<221>  primer_bind

<222>  (1)..(32)

<223>  primer

<400>  40
ttaggaattc ctaacactct cccctgttga ag                                     32

<210>  41

<211>  31

<212>  DNA

<213>  Artificial Sequence

<220>

<223>  5'-VH leader

<220>

<221>  primer_bind

<222>  (1)..(31)

<223>  primer

<400>  41
   aatatggatt ttgggctgat tttttttatt g                                    31

<210>  42

<211>  24

<212>  DNA

<213>  Artificial Sequence

<220>

<223>  3'-CH hinge

<220>

<221>  primer_bind

<222>  (1)..(24)

<223>  primer

<400>  42
aattgggcaa cgttgcaggt gacg                                              24

<210>  43

<211>  663

<212>  DNA

<213>  Mus musculus

<220>

<221>  misc_binding

<222>  (1)..(663)

<223>  DNA variable part of heavy chain 3A6

<400>  43
atggattttg ggctgatttt ttttattgtt ggtcttttaa aaggggtcca gtgtgaggtg      60

aagcttctcg agtctggagg tggcctggtg cagcctggag gatccctgaa actctcctgt     120

gtagtctcag gattcgattt tagaagaaat tggatgagtt gggtccggca ggctcctggg     180

aaagggctag aatggattgg agaaattaat ccagatagca gtaagataaa ctatacgcca     240

tctctaaagg ataaattcat catctccaga gacaatgcca agaatacgct gtacctgcaa     300

gtgagcacag tgagatctga ggacacagcc ctttattact gtgtgagacc ggtctggatg     360

tatgctatgg actactgggg tcaaggaacc tcagtcaccg tctcctcagc caaaacgaca     420

cccccatctg tctatccact ggcccctgga tctgctgccc aaactaactc catggtgacc     480

ctgggatgcc tggtcaaggg ctatttccct gagccagtga cagtgacctg gaactctgga     540

tccctgtcca gcggtgtgca caccttccca gctgtcctgc agtctgacct ctacactctg     600

agcagctcag tgactgtccc ctccagcacc tggcccagcg agaccgtcac ctgcaacgtt 660

gcc 663

<210> 44

<211> 717

<212> DNA

<213> Mus musculus

<220>

<221> misc_binding

<222> (1)..(717)

<223> variable part of light chain of 3A6

<400> 44
atgagtcctg cccagttcct gtttctgtta gtgctctgga ttcgggaaac cagcggtgat 60

gttctgttga cccagactcc tctcactttg tcgataacca ttggacaacc agcctccatc 120

tcttgcaagt caagtcagag cctcttgcat agtgatggaa agacatattt gaattggttg 180

ttacagaggc caggccagtc tccaaagcgc ctaatctatc tggtgtctaa actggactct 240

ggagtccctg acaggttcac tggcagtgga tcagggacgg atttcacact gaaaatcagc 300

agagtggagg ctgaggattt gggactttat tattgctggc aaggtacaca ttttcctcag 360

acgttcggtg gaggcaccaa gctggaaatc aaacgggctg atgctgcacc aactgtatcc 420

atcttcccac catccagtga gcagttaaca tctggaggtg cctcagtcgt gtgcttcttg 480

aacaacttct accccaaaga catcaatgtc aagtggaaga ttgatggcag tgaacgacaa 540

aatggcgtcc tgaacagttg gactgatcag gacagcaaag acagcaccta cagcatgagc 600

agcaccctca cgttgaccaa ggacgagtat gaacgacata acagctatac ctgtgaggcc 660

actcacaaga catcaacttc acccattgtc aagagcttca acaggggaga gtgttag 717